# EUROPEAN PATENT APPLICATION

(11) **EP 3 995 215 A1**
(43) Date of publication of application: **11.05.2022**
(21) Application number: 20834808.6
(22) Date of filing: 18.06.2020
(51) Int. Cl.: B01L 9/06, A61F 9/007

(54) **REAGENT HOLDER, TESTING APPARATUS ASSEMBLY, AQUEOUS HUMOR COLLECTION DEVICE AND AQUEOUS HUMOR COLLECTION METHOD**

(30) Priority: 02.07.2019 CN 201910591368; 02.07.2019 CN 201921024358 U; 02.07.2019 WO PCT/CN2019/094383; 02.07.2019 CN 201910591476; 02.07.2019 CN 201921028861 U; 02.07.2019 CN 201921024325 U; 02.07.2019 CN 201910590699; 02.07.2019 WO PCT/CN2019/094384; 09.10.2019 WO PCT/CN2019/110062; 24.04.2020 CN 202020647300 U; 24.04.2020 CN 202020644381 U
(71) Applicant: Beijing Sightnovo Medical Technology Co., Ltd, Beijing 100000 (CN)
(72) Inventor: XIA, Chaoran, Beijing 100000 (CN); SUN, Yueguang, Beijing 100039 (CN)
(74) Representative: Kilner, Matthew Simon
(86) International application number: PCT/CN2020/096860
(87) International publication number: WO 2021/000743

(57) **Abstract**

Disclosed are a reagent holder, a testing apparatus assembly, an aqueous humor collection device and an aqueous humor collection method. The reagent holder (9) comprises a holder body and a functional station arranged on the holder body, wherein the functional station comprises a fluid holding tube insertion hole (91) and an elastic component. The elastic component can elastically act on a fluid holding tube (106) in the case where the fluid holding tube (106) is inserted into the liquid holding tube insertion hole (91) so as to enable the fluid holding tube (106) to elastically move in the direction of the central axis of the hole of the fluid holding tube insertion hole (91). By means of using the reagent holder to elastically fix the fluid holding tube (106), the rigid collision between a fluid drawing device and a bottom wall of the fluid holding tube (106) can be converted into an elastic collision, so as to prevent a testing apparatus from triggering a firing pin protection function during the fluid drawing process, such that the depth to which the fluid drawing device can extend into the tube cavity of the fluid holding tube (106) is greatly increased, and the sample extraction amount is thus effectively increased.

## Description

### Technical Field

The invention relates to the technical field of medical appliances, in particular to an aqueous humor collection device and method, a reagent holder and testing apparatus assembly and an aqueous humor collection and testing combined device and method.

### Background

In the field of IVD (in vitro diagnostic products) at present, some testing apparatus are provided with a liquid-drawing needle to extend into a cavity of a sample container for sampling, and in order to prevent collision damage of the liquid-drawing needle, the testing apparatus are generally provided with a striker protection function, i.e. reporting an error and stopping when the needle head collides rigidly against the inner wall of the sample container. Therefore, to ensure the consistency of the sample drawing process, the penetration depth of the needle needs to be limited to prevent it from touching the bottom wall of the sample container. However, when the sample container can hold a small amount of sample, the needle can be inserted into the container to a shallow depth, which results in the sample being extracted in an excessively small amount.

### Disclosure of Invention

In view of the above-mentioned defects or shortcomings of the prior art, the present invention provides a reagent holder, a testing apparatus assembly, an aqueous humor collection device and an aqueous humor collection method, which can convert the rigid collision between a liquid drawing device and a fluid holding tube into elastic collision, and avoid triggering a striker protection function during the fluid drawing process to effectively increase the sample extraction amount.

To achieve the above object, in some embodiments, the present invention provides a reagent holder, which comprises a holder body and a functional station disposed on the holder body, wherein the functional station comprises a fluid holding tube insertion hole and an elastic component, and the elastic component is configured to: under the condition that a fluid holding tube is inserted into the fluid holding tube insertion hole, the elastic force can act on the fluid holding tube, so that the fluid holding tube can elastically move along the direction of the central axis of the hole of the fluid holding tube insertion hole.

Optionally, the functional station further comprises a functional station outer sleeve, the functional station outer sleeve extends downwards from the inner peripheral wall of the fluid holding tube insertion hole along the direction of the central axis of the hole, and the elastic component is located in a sleeve inner cavity of the functional station outer sleeve.

Optionally, the elastic component is a spring mechanism or an elastic medium.

Optionally, the spring mechanism is located at the bottom of the inner cavity of the sleeve and can elastically move along the central axis of the hole, and during the elastic movement of the spring mechanism, at least part of the outer peripheral part of the spring mechanism slides along the inner peripheral wall of the inner cavity of the sleeve.

Optionally, the reagent holder further comprises a conversion socket which is detachably inserted into the fluid holding tube insertion hole, the conversion socket comprises a support sleeve part and an extension sleeve part which are connected up and down, sleeve cavities of the support sleeve part and the extension sleeve part are communicated up and down and jointly form a conversion insertion cavity, and the conversion insertion cavity is used for detachably inserting the fluid holding tube. Optionally, the functional station is a sample station, a reagent station, a light measuring station, a reaction station or a reserved station.

In addition, in some embodiments, the invention provides a testing apparatus assembly, which comprises a testing apparatus, a fluid holding tube and the reagent holder, wherein the fluid holding tube is detachably inserted into the fluid holding tube insertion hole, and the testing apparatus comprises a liquid drawing device which is arranged to extend into a tube cavity of the fluid holding tube to draw liquid.

Optionally, the testing apparatus is arranged to stop when the liquid drawing device is subjected to a rigid impact.

Optionally, the functional station still includes the functional station outer sleeve, the functional station outer sleeve extends downwards from the inner peripheral wall of the fluid holding tube insertion hole along the direction of the central axis of the hole, the bottom outline in fluid holding tube is the back taper, fluid holding tube passes fluid holding tube insertion hole is in order to insert extremely the sleeve inner chamber of functional station outer sleeve just the top in fluid holding tube is not higher than the drill way in fluid holding tube insertion hole, the mouth of tube external diameter in fluid holding tube with the internal diameter of sleeve inner chamber is the same. Optionally, the fluid drawing device is provided with a liquid drawing needle for extending into the tube cavity of the fluid holding tube to draw liquid, the outer end face of the liquid drawing needle is formed into an inclined end face, and a liquid drawing port is formed on the inclined end face.

In the invention, when the fluid drawing device extends into the tube cavity of the fluid holding tube to draw liquid and touches the bottom wall of the fluid holding tube, the fluid holding tube can elastically move along the central axis direction of the hole of the fluid holding tube insertion hole under the elastic action of the elastic component in the reagent holder, so that the rigid collision of the fluid drawing device and the bottom wall of the fluid holding tube is converted into elastic collision, and under the elastic collision condition, the testing apparatus cannot trigger the firing pin protection function. In other words, under the condition that the reagent holder is adopted to elastically fix the fluid holding tube, the depth of the fluid drawing device extending into the tube cavity of the fluid holding tube is greatly increased, so that the sample extraction amount can be effectively increased. In some embodiments, the present invention provides a testing apparatus assembly comprising:
the reagent holder is provided with a holder body and a functional station arranged on the holder body,
and the functional station comprises a fluid holding tube insertion hole and an elastic component;
the fluid holding tube is detachably inserted into the fluid holding tube insertion hole and can elastically move along the direction of the central axis of the hole of the fluid holding tube insertion hole under the elastic action of the elastic component;
the testing apparatus is provided with a liquid drawing needle which is used for extending into the tube cavity of the fluid holding tube to draw liquid, the outer end face of the liquid drawing needle is formed into an inclined end face, and a liquid drawing port is formed in the inclined end face.

Optionally, the liquid drawing needle extends along a straight line and comprises a needle body section and a needle head section which are connected with each other, the outer end of the needle head section is provided with the fluid drawing port, and the central axis of the needle body section is intersected with the central axis of the fluid drawing port.

Optionally, an acute included angle formed by intersection of the central axis of the needle body section and the central axis of the fluid drawing port is α, and α satisfies: 0<α≤0.1°.

Optionally, the inclined end surface is a curved surface or a flat surface.

Optionally, the bottom wall in the cavity of the fluid holding tube is a plane wall or a curved wall. Optionally, the testing apparatus is arranged to stop when the liquid drawing needle is rigidly impacted.

Optionally, the functional station further comprises a functional station outer sleeve, the functional station outer sleeve extends downwards from the inner peripheral wall of the fluid holding tube insertion hole along the direction of the central axis of the hole, and the elastic component is located in a sleeve inner cavity of the functional station outer sleeve.

Optionally, the elastic component is a spring mechanism or an elastic medium.

Optionally, the elastic component is located the bottom of sleeve inner chamber, the top center of elastic component is formed with the support concave position that is used for supporting the bottom of fluid holding tube, the bottom outline of fluid holding tube is the back taper, the shape that supports concave position match in the bottom outline of fluid holding tube.

Optionally, the functional station is a sample station, a reagent station, a light measuring station, a reaction station or a reserved station.

In some embodiments, according to the above technical solution, since the fluid holding tube is elastically fixed in the reagent holder, when the liquid drawing needle extends into the lumen of the fluid holding tube to draw fluid and touches the bottom wall of the fluid holding tube, the liquid drawing needle and the bottom wall of the fluid holding tube only elastically collide with each other, and thus the striker protection function is not triggered. And because the outer terminal surface of the liquid drawing needle forms to slope terminal surface and the fluid drawing port set up on this slope terminal surface, even the liquid drawing needle touch the diapire of the fluid holding tube with different shapes, the fluid drawing port also cannot be sealed and cannot influence normally drawing fluid, this is favorable to increasing the sample extraction amount when drawing fluid, and it can guarantee drawing fluid smoothly in the process of drawing fluid.

In some embodiments, the reagent holder and testing apparatus assemblies of the present invention can be used in conjunction with aqueous humor collection devices and methods. The anterior chamber puncture of eyeball is a medical technology which punctures the cornea of eyeball through an instrument to cause the outflow of aqueous humor, has important application value in the clinical scientific research work of ophthalmology, and is mainly applied to intraocular pressure reduction, aqueous humor collection and other therapeutic anterior chamber drainage.

Currently, there is no specific instrument and consumable for performing the anterior chamber puncture, and a 1ml syringe needle is often used as the anterior chamber puncture needle in the actual clinical work. However, in the process of puncturing by using the 1ml syringe needle, the drainage speed of aqueous humor is difficult to control, when the drainage speed is too high, the intraocular pressure can be rapidly reduced, and in more serious cases, other complications can be caused. In addition, because after aqueous humor gathers and finishes, will further shift the aqueous humor in the syringe cavity to the micro-centrifuge tube, consequently at this in-process, can cause the waste of aqueous humor to and the existence is because aqueous humor contacts and receives the risk of polluting with external environment in the transfer process, thereby leads to the testing result to have the deviation, and the reliability is relatively poor. Therefore, it is necessary to develop a new collecting device that can solve the problems of poor safety and reliability during the anterior chamber puncture.

In view of the above-mentioned defects or shortcomings of the prior art, the present invention provides an aqueous humor collection device, which can control the drainage speed of aqueous humor during the puncturing process, and can prevent the aqueous humor from being polluted and wasted due to transfer after the aqueous humor is collected, thereby improving safety and reliability.

To achieve the above objects, in some embodiments, the present invention provides an aqueous humor collection device comprising:
the outer cylinder is provided with a puncture needle mounting end and a fluid collection tube mounting end which are positioned at two axial ends;
the puncture needle is fixedly arranged at the puncture needle mounting end and is provided with a puncture needle inner end positioned in the outer cylinder cavity and a puncture needle outer end positioned outside the outer cylinder cavity;
the fluid collection tube assembly comprises a fluid collection tube which is inserted into the outer cylinder cavity from the fluid collection tube mounting end and can be inserted, pulled and moved along the axial direction, and a stopcock which is arranged in the outer cylinder cavity and is used for sealing the tube orifice of the fluid collection tube;
wherein, the coefficient of static friction between stopcock and the inner circumferential wall of outer cylinder is µ1, the stopcock with the coefficient of static friction between stopcock and the port of fluid collection tube is µ2, and satisfies: µ1 <µ2;
or the stopcock is arranged at intervals on the inner peripheral wall of the outer cylinder.

Optionally, the annular limiting member is an elastic component.

Optionally, smooth contact is formed between the stopcock and the inner peripheral wall of the outer cylinder.

Optionally, the aqueous humor collection device further comprises an anti-shaking structure for limiting the radial displacement of the fluid collection tube assembly along the cylindrical cavity of the outer cylinder.

Optionally, the anti-shaking structure comprises an annular groove and an annular limiting member, the annular groove is formed in the outer circumferential wall of the fluid collection tube assembly, the annular limiting member is arranged circumscribing the annular groove, and the radially outer end of the annular limiting member abuts against the inner circumferential wall of the outer cylinder. Optionally, the annular limiting member is a rubber ring.

Optionally, the groove side wall of the annular groove is formed with a groove vent hole which is through along the axial direction of the outer cylinder cavity, and the outer cylinder cavity is communicated with the groove vent hole in the cylinder cavity area between the stopcock and the puncture needle mounting end.

Optionally, the fluid collection tube assembly includes supporting tube rack, the fluid collection tube is inserted into the inner cavity of the supporting tube rack, and the annular groove is provided on the outer peripheral wall of the supporting tube rack.

Optionally, the fluid collection tube assembly still includes supporting tube rack, the fluid collection tube is inserted into the inner cavity of the supporting tube rack, the anti-shaking structure includes a limiting protrusion protruding from the outer peripheral wall of the supporting tube rack or from the inner peripheral wall of the outer cylinder, and the radial ends of the limiting protrusion are respectively connected to the outer peripheral wall of the supporting tube rack and the outer peripheral wall of the outer cylinder.

Optionally, the aqueous humor collection device further comprises a needle seat for fixedly mounting the puncture needle, and the inner end of the needle seat is connected with the mounting end of the puncture needle in a quick plugging manner.

Optionally, a threaded insertion or a buckling insertion is formed between the inner end of the needle seat and the mounting end of the puncture needle.

Optionally, the aqueous humor collection device further comprises an anti-puncture spacing piece for isolating the inner end of the puncture needle from the stopcock along the axial direction of the cylindrical cavity of the outer cylinder, and the anti-puncture spacing piece is a detachable piece. Optionally, the peripheral wall of the outer cylinder is provided with a spacer inserting groove, and the cylinder cavity of the outer cylinder communicates with the spacer inserting groove in the area between the inner end of the puncture needle and the stopcock, the anti-puncture spacing piece is inserted into the cylinder cavity area through the spacer inserting groove.

In some embodiments, the fluid collection tube assembly makes the fluid collection tube lumen be in the negative pressure state owing to be equipped with the stopcock, when the puncture needle inner punctures the stopcock in order to drainage aqueous humor to the fluid collection tube lumen, along with the liquid volume in the lumen increases gradually, the difference between tube lumen pressure and anterior chamber pressure reduces gradually, consequently makes the drainage speed of aqueous humor reduce gradually to avoid appearing the dangerous condition of intraocular pressure slump, improve the security greatly. In addition, because it is defined that the static friction coefficient µ1 between stopcock and the inner peripheral wall of the outer cylinder is smaller than the static friction coefficient µ2 between stopcock and the fluid collection tube port, or the stopcock is arranged on the inner peripheral wall of the outer cylinder, to ensure that when the extracting fluid collection tube assembly from outer cylinder chamber, the stopcock blocks up the fluid collection tube port tightly all the time, avoids aqueous humor and external environment in the lumen to contact, guarantees the pure degree of the aqueous humor of gathering, and can avoid owing to shift the waste that the aqueous humor caused to improve the reliability of follow-up testing.

In some embodiments, the present invention provides an aqueous humor collection device comprising: the outer cylinder is provided with a puncture needle mounting end and a fluid collection tube mounting end;
the puncture needle is inserted into the puncture needle mounting end and comprises a puncture needle outer end and a puncture needle inner end extending into the cylinder cavity of the outer cylinder;
the fluid collection tube assembly is inserted into the outer cylinder cavity from the fluid collection tube mounting end in a pluggable and movable manner and comprises a fluid collection tube and a stopcock used for sealing the tube orifice of the fluid collection tube in the outer cylinder cavity; wherein, a ventilation structure is formed between the fluid collection tube assembly and the inner peripheral wall of the outer cylinder, and the ventilation structure communicates with the cylinder cavity area of the outer cylinder cavity between the inner end of the puncture needle and the stopcock,
the aqueous humor collection device further includes a limiting buffer structure for limiting the displacement of the fluid collection tube assembly in the radial direction and for forming contact damping between the fluid collection tube assembly and the inner peripheral wall of the outer cylinder.

Optionally, the limiting buffer structure comprises a limiting protrusion protruding from the outer circumferential wall of the fluid collection tube assembly or from the inner circumferential wall of the outer cylinder, and two radial ends of the limiting protrusion are respectively connected with the outer circumferential wall of the fluid collection tube assembly and the inner circumferential wall of the outer cylinder.

Optionally, the limiting buffer structure comprises a limiting protrusion follows the tube length direction of the fluid collection tube assembly sets at intervals or follows the tube length direction extends, the total span of the limiting protrusion followed tube length direction is not less than the half of fluid collection tube.

Optionally, the limiting protrusion is integrally formed on the outer peripheral wall of the fluid collection tube; or, the fluid collection tube assembly includes that the overcoat is in the tube kit on the periphery wall of fluid collection tube, the limiting protrusion sets up on the periphery wall of tube kit.

Optionally, the tube kit is an elastic tube kit.

Optionally, the limiting buffer structure comprises a plurality of limiting protrusion which are sequentially arranged at intervals along the circumferential direction, and gaps among the limiting protrusion formed the ventilation structure;
or, the limiting buffer structure includes along the continuous shaping of extending of circumference the limiting protrusion, the limiting protrusion with be formed with between the periphery wall of fluid collection tube assembly or the limiting protrusion with be formed with between the inner peripheral wall of the cylinder outward as ventilation structure's exhaust clearance.

Optionally, the plurality of limiting protrusions sequentially arranged at intervals along the circumferential direction are all in a convex point shape. Optionally, the limiting protrusions which are formed by extending continuously along the circumferential direction are corrugated or helical teeth. Optionally, the fluid collection tube assembly still includes the supporting tube rack that is used for fixed mounting the fluid collection tube, the limiting buffer structure is formed between the outer peripheral wall of the supporting tube rack and the inner peripheral wall of the outer cylinder. Optionally, the fluid collection tube and the supporting tube rack are fixed by screw connection or snap connection.

In some embodiments, since the aqueous humor collection device is provided with a limit buffer structure, it can limit its displacement along the radial direction during the process of plugging and unplugging the fluid collection tube assembly, that is, to ensure that the fluid collection tube assembly only moves in the axial direction, and it can produce a certain degree of movement damping to prevent the insertion and removal speed from being too fast, so it can not only ensure that the inner end of the puncture needle accurately pierces the stopcock to be inserted into the lumen of the fluid collection tube, but also ensures that it can be pulled out stably after the aqueous humor is collected. The liquid extraction tube assembly is taken out to avoid the leakage of aqueous humor in the tube cavity and greatly improve the reliability. In addition, the fluid collection tube assembly is provided with a stopcock so that the fluid collection tube lumen is in a negative pressure state. When the inner end of the puncture needle pierces the stopcock to drain the aqueous humor to the fluid collection tube lumen, it follows the amount of fluid gradually increases, and the difference between the pressure in the lumen and the pressure in the anterior chamber is gradually reduced, so that the drainage speed of aqueous humor is gradually reduced, so as to avoid the dangerous situation of sudden drop in intraocular pressure and greatly improve safety. Furthermore, after the collection is completed, since the fluid collection tube assembly can be pulled out from the outer cylinder cavity as a whole, and the aqueous humor in the fluid collection tube can be detected directly, no further transfer is required, so it can avoid the aqueous humor in contact with the external environment to ensure the purity of the collected aqueous humor, thereby improving the accuracy of subsequent testing.

In some embodiments, the present invention provides an aqueous humor collection device comprising:
the outer cylinder comprises a puncture needle mounting end and a fluid collection tube mounting end;
the puncture needle is fixedly arranged at the puncture needle mounting end and comprises a puncture needle inner end and a puncture needle outer end which respectively extend out of two axial sides of the puncture needle mounting end;
the fluid collection tube assembly comprises a fluid collection tube which is inserted into the cavity of the outer cylinder from the fluid collection tube mounting end and can be inserted, pulled and moved along the axial direction, and a stopcock which is arranged in the cavity of the outer cylinder and is used for sealing the tube orifice of the fluid collection tube;
wherein, the stopcock is arranged coaxially with the cavity of the outer cylinder, and the radius of the stopcock is smaller than the radius of the inner peripheral wall of the outer cylinder, and the outer peripheral wall of the fluid collection tube assembly is provided with an annular groove and an annular limiting member, the annular limiting member is arranged circumscribing the annular groove, and the radially outer end of the annular limiting member abuts against the inner circumferential wall of the outer cylinder.

Optionally, a radial distance between a radially outer end of the stopcock and the inner peripheral wall of the outer cylinder is not less than 1 mm.

Optionally, the fluid collection tube assembly includes a supporting tube rack, the fluid collection tube is inserted into the inner cavity of the supporting tube rack, and the annular groove is provided on the outer peripheral wall of the supporting tube rack.

Optionally, a threaded connection or a snap connection is formed between the fluid collection tube and the supporting tube rack.

Optionally, the groove side wall of the annular groove is formed with a groove vent hole penetrating along the axial direction of the outer cylinder cavity, and the outer cylinder cavity is between the stopcock and the piercing hole. The cylinder cavity area between the needle mounting ends communicates with the groove vent.

Optionally, the annular limiting member is an elastic component.

Optionally, the aqueous humor collection device further comprises a needle seat for fixedly mounting the puncture needle and the inner end of the needle seat is connected with the mounting end of the puncture needle in a quick plugging manner.

Optionally, a threaded insertion or a buckling insertion is formed between the inner end of the needle seat and the mounting end of the puncture needle.

Optionally, the aqueous humor collection device further comprises an anti-puncture spacing piece for isolating the inner end of the puncture needle from the stopcock along the axial direction of the cylindrical cavity of the outer cylinder, and the anti-puncture spacing piece is a detachable piece. Optionally, the peripheral wall of the outer cylinder is provided with a spacer inserting groove, and the cylinder cavity of the outer cylinder communicates with the spacer inserting groove in the area between the inner end of the puncture needle and the stopcock, the anti-puncture spacing piece is inserted into the cylinder cavity area through the spacer inserting groove.

In some embodiments, the fluid collection tube assembly makes the fluid collection tube lumen be in the negative pressure state owing to be equipped with the stopcock, when the puncture needle inner punctures the stopcock in order to drainage aqueous humor to the fluid collection tube lumen, along with the liquid volume in the lumen increases gradually, the difference between tube lumen pressure and anterior chamber pressure reduces gradually, consequently makes the drainage speed of aqueous humor reduce gradually to avoid appearing the dangerous condition of intraocular pressure slump, improve the security greatly. In addition, because it is defined that the radius of stopcock be less than the radius of the inner peripheral wall of the outer cylinder, consequently stopcock and the inner peripheral wall of the outer cylinder do not produce the contact, to ensure that when the extracting fluid collection tube assembly from the outer cylinder, the stopcock blocks up the fluid collection tube port tightly all the time, avoids aqueous humor and external environment in the lumen to contact, guarantees the pure degree of the aqueous humor of gathering, and can avoid owing to shift the waste that the aqueous humor caused to improve the reliability of follow-up testing.

In some embodiments, the present invention provides an aqueous humor collection device comprising:
the outer cylinder comprises a puncture needle mounting end and a fluid collection tube mounting end;
the puncture needle is fixedly arranged at the puncture needle mounting end and is provided with a puncture needle inner end positioned in the outer cylinder cavity and a puncture needle outer end positioned outside the outer cylinder cavity;
the fluid collection tube assembly comprises a fluid collection tube which is inserted into the cavity of the outer cylinder from the fluid collection tube mounting end and can be inserted, pulled and moved along the axial direction, and a stopcock which is arranged in the cavity of the outer cylinder and is used for sealing the tube orifice of the fluid collection tube;
wherein, the outer peripheral wall of the fluid collection tube assembly is provided with an annular groove and an annular limiting member, the annular limiting member is arranged circumscribing the annular groove, and the radially outer end of the annular limiting member abuts against the inner circumferential wall of the outer cylinder, the radial outer marginal part of stopcock forms smooth contact with the inner peripheral wall of the outer cylinder and be equipped with stopcock exhaust passage that the extending direction in outer cylinder set up, the cylinder cavity of the outer cylinder communicates with the stopcock exhaust passage in the cylinder cavity area between the stopcock and the puncture needle mounting end.

Optionally, the stopcock is provided with a plurality of stopcock exhaust passages which extend along the axial direction of the outer cylinder cavity and are sequentially arranged at intervals along the circumferential direction of the stopcock.

Alternatively, a stopcock exhaust groove provided with a groove opening facing radially outward is formed on the outer peripheral wall of the stopcock as the stopcock exhaust passage.

Optionally, the channel peripheral wall of the stopcock exhaust passage is a closed peripheral wall. Optionally, the stopcock exhaust passage has a cross-section in the shape of a semicircle, a square, or an arc.

Optionally, the stopcock comprises a stopcock central portion for sealing the tube orifice of the fluid collection tube and a stopcock outer ring portion located radially outside the stopcock central portion and provided with the stopcock exhaust passage, and the stopcock outer ring portion is at least partially formed into a loose ventilation structure.

Optionally, a puncture guide blind hole for guiding the inner end of the puncture needle to penetrate is formed in the end portion of the stopcock, and the outer contour shape of the inner end of the puncture needle is matched with the shape of the puncture guide blind hole.

Optionally, a peripheral wall exhaust gap is arranged between the fluid collection tube and the inner peripheral wall of the outer cylinder, and the cylinder cavity of the outer cylinder is sequentially communicated with the cylinder cavity area between the stopcock and the puncture needle mounting end, the stopcock exhaust passage and the peripheral wall exhaust gap.

Optionally, a peripheral wall water outlet hole communicated with the needle cavity of the puncture needle is formed in the peripheral wall of the inner end of the puncture needle.

Optionally, the inner end of the puncture needle is further provided with an axial water outlet hole axially communicated with the needle cavity, and the axial distance between the hole center of the peripheral wall water outlet hole and the hole center of the axial water outlet hole is not more than 3 mm.

In some embodiments, the stopcock exhaust passage is arranged at the radial outer edge part of the stopcock, so that the atmospheric environment can be communicated with the cylinder cavity area of the cylinder cavity of the outer cylinder between the stopcock and the puncture needle mounting end, the air plug is prevented from being formed in the cylinder cavity of the outer cylinder, the smooth plugging and unplugging of the fluid collection tube assembly is prevented from being influenced, and the use reliability is improved. In addition, at the drainage in-process, the fluid collection tube assembly makes the fluid collection tube lumen be in the negative pressure state owing to be equipped with the stopcock, when the inner end of the puncture needle pierces the stopcock to drain the aqueous humor to the lumen of the fluid collection tube, as the amount of fluid in the lumen gradually increases, the difference between the pressure in the lumen and the pressure in the anterior chamber gradually decreases, therefore, the drainage speed of aqueous humor is gradually reduced, thereby avoiding the dangerous situation of sudden drop in intraocular pressure and greatly improving safety. Furthermore, after the collection is completed, the fluid collection tube assembly can be pulled out from the outer cylinder cavity as a whole, and the aqueous humor in the fluid collection tube can be directly detected without further transfer, therefore, the aqueous humor in the lumen can be prevented from contacting the external environment, and the purity of the collected aqueous humor can be ensured, thereby improving the accuracy of subsequent detection.

In some embodiments, the present invention provides an aqueous humor collection device comprising: the outer cylinder comprises a puncture needle mounting end and a fluid collection tube mounting end;
the puncture needle is fixedly arranged at the puncture needle mounting end and is provided with a puncture needle inner end positioned in the outer cylinder cavity and a puncture needle outer end positioned outside the outer cylinder cavity;
the fluid collection tube is inserted into the outer cylinder cavity from the fluid collection tube mounting end and can be inserted, pulled and moved along the axial direction;
a stopcock is arranged in the cylinder cavity of the outer cylinder and used to seal the nozzle of the fluid collection tube, the radially outer edge of the stopcock is provided with a stopcock exhaust passage arranged along the extension direction of the outer cylinder cavity, and the stopcock exhaust passage communicates with the area of the cylinder cavity between the mounting end of the puncture needle and the stopcock in the outer cylinder cavity.

Optionally, the stopcock is provided with a plurality of stopcock exhaust passages which extend along the axial direction of the outer cylinder cavity and are sequentially arranged at intervals along the circumferential direction of the stopcock.

Alternatively, a stopcock exhaust groove serving as the stopcock exhaust passage is formed on the outer peripheral wall of the stopcock, and the stopcock exhaust groove is provided with a groove opening toward the radially outer side.

Optionally, the channel peripheral wall of the stopcock exhaust passage is a closed peripheral wall. Optionally, the stopcock exhaust passage has a cross-section in the shape of a semicircle, a square, or an arc.

Optionally, the stopcock comprises a stopcock central portion for sealing the tube orifice of the fluid collection tube and a stopcock outer ring portion located radially outside the stopcock central portion and provided with the stopcock exhaust passage, and the stopcock outer ring portion is at least partially formed into a loose ventilation structure.

Optionally, a puncture guide blind hole for guiding the inner end of the puncture needle to penetrate is formed in the end portion of the stopcock, and the outer contour shape of the inner end of the puncture needle is matched with the shape of the puncture guide blind hole.

Optionally, a peripheral wall exhaust gap is arranged between the fluid collection tube and the inner peripheral wall of the outer cylinder, and the cylinder cavity of the outer cylinder is sequentially communicated with the cylinder cavity area between the stopcock and the puncture needle mounting end, the stopcock exhaust passage and the peripheral wall exhaust gap.

Optionally, a peripheral wall water outlet hole communicated with the needle cavity of the puncture needle is formed in the peripheral wall of the inner end of the puncture needle.

Optionally, the inner end of the puncture needle is further provided with an axial water outlet hole axially communicated with the needle cavity, and the axial distance between the hole center of the peripheral wall water outlet hole and the hole center of the axial water outlet hole is not more than 3 mm.

In some embodiments, the stopcock exhaust passage is arranged at the radial outer edge part of the stopcock, so that the atmospheric environment can be communicated with the cylinder cavity area of the cylinder cavity of the outer cylinder between the stopcock and the puncture needle mounting end, the air plug is prevented from being formed in the cylinder cavity of the outer cylinder, the smooth plugging and unplugging of the fluid collection tube assembly is prevented from being influenced, and the use reliability is improved. In addition, at the drainage in-process,
the fluid collection tube assembly makes the fluid collection tube lumen be in the negative pressure state owing to be equipped with the stopcock, when the puncture needle inner punctures the stopcock
in order to drainage aqueous humor to the fluid collection tube lumen, along with the liquid volume in the lumen increases gradually, the difference between tube lumen pressure and anterior chamber pressure reduces gradually, consequently makes the drainage speed of aqueous humor reduce gradually to avoid appearing the dangerous condition of intraocular pressure slump, improve the security greatly. Moreover, after the collection finishes, can wholly extract the fluid collection tube from outer cylinder to directly detect the aqueous humor in the fluid collection tube, no further transfer is required, so it can avoid the aqueous humor in contact with the external environment to ensure the purity of the collected aqueous humor, thereby improving the accuracy of subsequent testing.

In some embodiments, the invention provides an aqueous humor collection device, which comprises an outer cylinder, a puncture needle and a fluid collection tube assembly, wherein the outer cylinder is provided with a puncture needle mounting end and a fluid collection tube mounting end, the puncture needle is inserted into the puncture needle mounting end and comprises an outer end of the puncture needle and an inner end of the puncture needle inserted into a cylinder cavity of the outer cylinder, and the fluid collection tube assembly is inserted into the cylinder cavity of the outer cylinder from the fluid collection tube mounting end and comprises a fluid collection tube capable of being plugged and moved and a stopcock used for sealing a tube orifice of the fluid collection tube in the cylinder cavity of the outer cylinder; the aqueous humor collection device further comprises an anti-shaking structure used for limiting the fluid collection tube assembly to move along the radial direction of the cylinder cavity of the outer cylinder.

Optionally, the fluid collection tube assembly still includes supporting tube rack, the fluid collection tube is inserted into the inner cavity of the supporting tube rack, the anti-shaking structure includes a limiting protrusion protruding from the outer peripheral wall of the supporting tube rack or from the inner peripheral wall of the outer cylinder, and the radial ends of the limiting protrusion are respectively connected to the outer peripheral wall of the supporting tube rack and the outer peripheral wall of the outer cylinder.

Optionally, the fluid collection tube and the supporting tube rack are fixed by screw connection or snap connection.

Optionally, the limiting protrusion is integrally formed on the outer peripheral wall of the fluid collection tube; or, the fluid collection tube assembly includes that the overcoat is in the tube kit on the periphery wall of fluid collection tube, the limiting protrusion sets up on the periphery wall of tube kit.

Optionally, the tube kit is an elastic tube kit.

Optionally, the anti-shaking structure comprises a plurality of limiting protrusion which are sequentially arranged at intervals along the circumferential direction;
or, the anti-shaking structure comprises a limiting protrusion which is formed by continuously extending along the circumferential direction, and an exhaust gap is formed between the limiting protrusion and the outer circumferential wall of the supporting tube rack or between the limiting protrusion and the inner circumferential wall of the outer cylinder.

Optionally, the plurality of limiting protrusions sequentially arranged at intervals along the circumferential direction are all in a convex point shape.

Optionally, the limiting protrusion which are formed by extending continuously along the circumferential direction are corrugated or helical teeth.

Optionally, the anti-shaking structure comprises limiting protrusions arranged at intervals in sequence along the tube length direction of the fluid collection tube assembly or continuously extending along the tube length direction, and the total span of the limiting protrusions along the tube length direction is not less than half of the tube length of the fluid collection tube.

In some embodiments, because aqueous humor collection system is equipped with anti-shaking structure, it can limit its displacement along the radial direction during the process of plugging and unplugging the fluid collection tube assembly, that is, to ensure that the fluid collection tube assembly only moves in the axial direction, so it can not only ensure that the inner end of the puncture needle accurately pierces the stopcock to be inserted into the lumen of the fluid collection tube, but also ensures that it can be pulled out stably after the aqueous humor is collected. The liquid extraction tube assembly is taken out to avoid the leakage of aqueous humor in the tube cavity and greatly improve the reliability. In addition, the fluid collection tube assembly is provided with a stopcock so that the fluid collection tube lumen is in a negative pressure state. When the inner end of the puncture needle pierces the stopcock to drain the aqueous humor to the fluid collection tube lumen, it follows the amount of fluid gradually increases, and the difference between the pressure in the lumen and the pressure in the anterior chamber is gradually reduced, so that the drainage speed of aqueous humor is gradually reduced, so as to avoid the dangerous situation of sudden drop in intraocular pressure and greatly improve safety. Furthermore, after the collection is completed, since the fluid collection tube assembly can be pulled out from the outer cylinder cavity as a whole, and the aqueous humor in the fluid collection tube can be detected directly, no further transfer is required, so it can avoid the aqueous humor in contact with the external environment to ensure the purity of the collected aqueous humor, thereby improving the accuracy of subsequent testing.

In some embodiments, the present invention provides a method of collecting aqueous humor from a cornea, comprising:
the outer end of a puncture needle of the puncture needle of any aqueous humor collection device provided by the invention is punctured into the anterior chamber of an eye;
pushing the rear end of a fluid collection tube assembly to enable a fluid collection tube in the fluid collection tube assembly to move towards the inner end of the puncture needle, so that the inner end of the puncture needle penetrates through a stopcock and enters the fluid collection tube;
allowing aqueous humor of the anterior chamber to flow through the puncture needle and into the fluid collection tube at a pressure differential between the intraocular pressure and the fluid collection tube;
withdrawing the aqueous humor collection device from the anterior chamber; and taking out the fluid collection tube assembly from the outer cylinder.

Optionally, the method comprises pushing the rear end of the fluid collection tube assembly to move the fluid collection tube in the fluid collection tube assembly towards the inner end of the puncture needle to pass the inner end of the puncture needle through the stopcock and into the fluid collection tube. Optionally, the method comprises holding the aqueous humor collection device with a single hand and nudging the rear end of the fluid collection tube assembly with an index finger.

In some embodiments, the present invention provides a method for collecting aqueous humor from an eye of a subject, comprising inserting an outer end of a puncture needle of any of the aqueous humor collection devices provided according to the present invention into an anterior chamber of the eye, wherein a pressure within the fluid collection tube is lower than an intraocular pressure of the subject; pushing the rear end of a fluid collection tube assembly to enable a fluid collection tube in the fluid collection tube assembly to move towards the inner end of the puncture needle, so that the inner end of the puncture needle penetrates through a stopcock and enters the fluid collection tube;
allowing aqueous humor of the anterior chamber of the eye to flow through the puncture needle and into the fluid collection tube at a pressure differential between the intraocular pressure and the negative pressure;
withdrawing the aqueous humor collection device from the anterior chamber; and and taking out the fluid collection tube assembly from the outer cylinder.

Optionally, the method comprises pushing the rear end of the fluid collection tube assembly to move the fluid collection tube in the fluid collection tube assembly towards the inner end of the puncture needle to pass the inner end of the puncture needle through a stopcock into the fluid collection tube. Optionally, the method comprises holding the aqueous humor collection device with a single hand and nudging the rear end of the fluid collection tube assembly with an index finger.

Optionally, the method further comprises selecting a negative pressure inside the fluid collection tube that is below an intraocular pressure of an eye of the subject.

Additional features and advantages of the invention will be set forth in the detailed description which follows.

### Drawings

The accompanying drawings, which are included to provide a further understanding of the invention and are incorporated in and constitute a part of this specification, illustrate embodiments of the invention and together with the description serve to explain the principles of the invention and not to limit the invention. In the drawings:
FIG. 1 is a schematic view of a reagent holder in an embodiment of the present invention;
FIG. 2 is a schematic view of a spring mechanism in an embodiment of the present invention;
FIG. 3 is a schematic view of a testing apparatus in an embodiment of the present invention;
FIG. 4 is a schematic view of a fluid holding tube in an embodiment of the present invention;
FIG. 5 is a schematic view of a pipetting needle in accordance with an embodiment of the invention;
FIG. 6 is a perspective view of an aqueous humor collection device according to an embodiment of the present invention;
FIG. 7 is a front view of the aqueous humor collection device of FIG. 6;
FIG. 8 is a front cross-sectional view of the aqueous humor collection device of FIG. 7 without a puncture needle piercing the stopcock;
FIG. 9 is a front cross-sectional view of the aqueous humor collection device of FIG. 8 provided with a puncture-proof barrier;
FIG. 10 is a cross-sectional view of the aqueous humor collection device of FIG. 6;
FIG. 11 is a schematic structural view of the puncture needle of the aqueous humor collection device in FIG. 8, which is provided with peripheral wall water outlet hole;
fig. 12 is a schematic view of the stopcock of the aqueous humor collection device in fig. 8, which is provided with a stopcock exhaust passage having an open peripheral wall and a semicircular shape; fig. 13 is a schematic structural view of the stopcock of the aqueous humor collection device in fig. 8, which is provided with a stopcock exhaust passage with an open peripheral wall and a semi-rectangular shape;
fig. 14 is a schematic structural view of the stopcock of the aqueous humor collection device in fig. 8, wherein the stopcock is provided with a circular stopcock exhaust passage with a closed peripheral wall;
fig. 15 is a schematic structural view of the stopcock of the aqueous humor collection device in fig. 8, which is provided with a stopcock exhaust passage with a closed circumferential wall and an arc shape;
FIG. 16 is a schematic structural view of the aqueous humor collection device of FIG. 8 with the fluid collection tube and the supporting tube rack being connected by a snap fit;
fig. 17 is a schematic structural view of a fluid collection tube of the aqueous humor collection device according to the embodiment of the present invention, in which a plurality of convex limiting protrusions are integrally formed;
fig. 18 is a schematic structural view of a fluid collection tube of the aqueous humor collection device according to the embodiment of the present invention, in which a corrugated limiting protrusion is integrally formed;
fig. 19 is a schematic structural view of a fluid collection tube of the aqueous humor collection device according to the embodiment of the present invention, wherein a tube kit is sleeved outside the fluid collection tube, and the limiting protrusion is in the form of a convex point;
fig. 20 is a schematic structural view of a fluid collection tube of the aqueous humor collection device according to the embodiment of the present invention, wherein a tube kit is sleeved outside the fluid collection tube, and the limiting protrusions are corrugated;
fig. 21 is a schematic structural view of a fluid collection tube of the aqueous humor collection device according to the embodiment of the present invention, wherein a tube kit is sleeved outside the fluid collection tube, and the limiting protrusions are in the shape of helical teeth;
fig. 22 is a schematic structural view of a supporting tube rack of the aqueous humor collection device according to the embodiment of the present invention, in which the supporting tube rack is integrally formed with a plurality of protruding limiting protrusions;
fig. 23 is a schematic structural view of a supporting tube rack of the aqueous humor collection device according to the embodiment of the present invention, in which the supporting tube rack is integrally formed with a plurality of corrugated limiting protrusions;
fig. 24 is a schematic structural view of a supporting tube rack of the aqueous humor collection device according to the embodiment of the present invention, in which the supporting tube rack is integrally formed with a helical tooth-shaped limiting protrusion;
fig. 25 is a schematic structural view of a supporting tube rack of the aqueous humor collection device according to the embodiment of the present invention, wherein a tube kit is sleeved outside the supporting tube rack, and the limiting protrusions are protruded;
fig. 26 is a schematic structural view of a supporting tube rack of the aqueous humor collection device according to the embodiment of the present invention, wherein a tube kit is sleeved outside the supporting tube rack, and the limiting protrusions are corrugated;
fig. 27 is a schematic structural view of a supporting tube rack of the aqueous humor collection device according to the embodiment of the present invention, wherein a tube kit is sleeved outside the supporting tube rack, and the limiting protrusions are in the shape of helical teeth;
FIG. 28 is a schematic diagram of a switching jack according to an embodiment of the present invention;
FIG. 29 is a schematic diagram comparing absolute deviation from the target volume (ADTV) for each group of anterior aqueous humor collection and each group of target collection (50 µL) in an animal experiment in an embodiment of the present invention;
FIG. 30 is a schematic of the operating time of each subgroup in an animal experiment in an embodiment of the present invention;
FIG. 31 is a schematic representation of a front-end evaluation of representative group 1 rabbits in an animal experiment in an embodiment of the present invention.

**Description of the reference numerals:**

| | | | |
|---|---|---|---|
| 1 | outer cylinder | 2 | puncture needle |
| 3 | fluid collection tube assembly | 4 | needle seat |
| 5 | anti-puncture spacing piece | 6 | limiting protrusion |
| 7 | needle cap | 8 | soft sleeve |
| 11 | spacer inserting groove | 21 | peripheral wall water outlet hole |
| 22 | axial water outlet hole | 31 | fluid collection tube |
| 32 | supporting tube rack | 33 | stopcock |
| 34 | tube kit | 35 | annular groove |
| 36 | groove vent hole | 37 | annular limiting member |
| 311 | fluid collection tube clamping groove | 321 | tube rack fastener |
| 322 | tube rack button | 331 | stopcock exhaust passage |
| 332 | puncture guide blind hole | | |
| 9 | reagent holder | 10 | testing apparatus |
| 91 | fluid holding tube insertion hole | 92 | functional bit outer sleeve |
| 93 | spring mechanism | 94 | left anchoring piece |
| 95 | sample station | 96 | dilution station |
| 97 | washing solution station | 98 | reagent station |
| 99 | light measuring station | 910 | reaction station |
| 911 | substrate station | 912 | reserved station |
| 913 | right anchoring piece | 914 | switching jack |
| 101 | shell | 102 | reaction cabin |
| 103 | hatch | 104 | liquid drawing needle |
| 105 | reagent holder station | 106 | fluid holding tube |
| 93a | support recess | 914a | supporting sleeve part |
| 914b | extending sleeve part | 104a | inclined end face |
| 104b | needle body section central axis | 104c | liquid drawing port central axis |

### Detailed Description

The following describes in detail embodiments of the present invention with reference to the drawings. It should be understood that the detailed description and specific examples, while indicating embodiments of the invention, are given by way of illustration and explanation only, not limitation.

It should be noted that the embodiments and features of the embodiments of the present invention may be combined with each other without conflict.

In the embodiments of the present invention, unless otherwise specified, the use of directional terms such as "upper, lower, top, and bottom" generally refer to the orientation shown in the drawings or to the positional relationship of the components relative to each other in the vertical, or gravitational direction.

The invention will be described in detail hereinafter with reference to the drawings and in connection with exemplary embodiments.

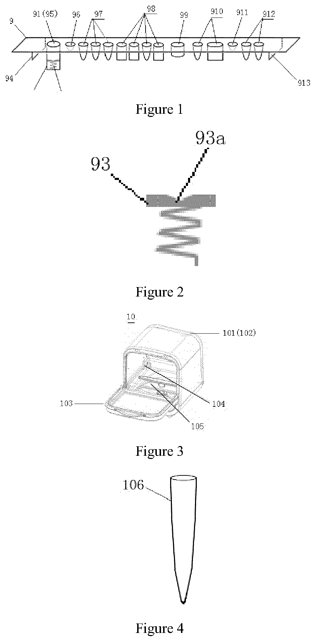

In some embodiments, such as shown in fig. 1-5, the present invention provides a reagent holder 9, and the reagent holder 9 can be configured as a strip supporting as shown. Wherein, reagent holder 9 includes the holder body and functional station provided on the holder body, and this functional station includes fluid holding tube insertion hole 91 and elastic component, and the elastic component is configured to: when the fluid holding tube 106 is inserted into the fluid holding tube insertion hole 91, the fluid holding tube 106 is elastically moved in the direction of the central axis of the fluid holding tube insertion hole 91 by elastically acting on the fluid holding tube 106.

Through setting up above-mentioned structure, when the fluid drawing device among the testing apparatus 10 stretched into the tube lumen of fluid holding tube 106 and got liquid and touched its diapire, fluid holding tube 106 can follow the hole central axis direction elastic movement of fluid holding tube insertion hole 91 owing to receive the elastic component's in the reagent holder 9 elastic reaction to with the rigid collision of the diapire of liquid drawing device and fluid holding tube 106 transform into elastic collision, under the elastic collision circumstances, check out test set cannot trigger firing pin protect function. In other words, in the case of elastically fixing the fluid holding tube 106 by using the reagent holder 9 of the present invention, the depth of the fluid drawing device extending into the cavity of the fluid holding tube 106 is greatly increased, so that the sample extraction amount can be effectively increased.

In one embodiment, the functional station further comprises a functional station outer sleeve 92, and the functional station outer sleeve 92 extends downward from the inner peripheral wall of the fluid holding tube insertion hole 91 in the direction of the central axis of the hole. In this case, the elastic component may be provided in the sleeve inner cavity of the functional bit outer sleeve 92, for example, at the bottom or the periphery of the sleeve inner cavity, as long as the fluid holding tube 106 is elastically movable in the direction of the hole center axis of the fluid holding tube insertion hole 91.

In one embodiment, the elastic component may be provided as a spring mechanism 93 or an elastic medium. When the elastic component is provided as an elastic medium, the elastic medium may be filled between the inner wall of the functional bit outer sleeve 92 and the outer wall of the fluid holding tube 106. When the elastic component is provided as the spring mechanism 93, referring to fig. 1 and fig. 2, the spring mechanism 93 may be disposed at the bottom of the sleeve inner cavity of the functional bit outer sleeve 92, and the spring mechanism 93 may be elastically movable along the hole central axis direction, and during the elastic movement of the spring mechanism 93, the outer circumferential portion of the spring mechanism 93 may be disposed to at least partially adhere to the inner circumferential wall of the sleeve inner cavity to slide, so as to ensure that the elastic force acting on the fluid holding tube 106 always follows the hole central axis direction of the fluid holding tube insertion hole 91.

In one embodiment, the elastic component may be provided as a spring mechanism 93 or an elastic medium. When the elastic component is set as an elastic medium, the elastic medium can be filled between the inner wall of the functional bit outer sleeve 92 and the outer wall of the fluid holding tube 106. When the elastic component is set as the spring mechanism 93, referring to Figures 1 and 2, the spring mechanism 93 can be set at the bottom of the sleeve cavity of the functional bit outer sleeve 92 at the functional station and the spring mechanism 93 can be elastically moved along the direction of the central axis of the hole, and during the elastic movement of the spring mechanism 93, the outer periphery of the spring mechanism 93 can be set to at least partially fit the inner peripheral wall of the sleeve cavity to slide, so as to ensure that the elastic force acting on the fluid holding tube 106 is always along the direction of the hole center axis of the fluid holding tube insertion hole 91.

In an embodiment, when the elastic component is arranged at the bottom of the functional bit outer sleeve 92, a support recess 93a may be provided in the center of the top of the elastic component, and the support recess 93a is used to support the bottom of the fluid holding tube 106. For example, referring to FIG. 4, the outer contour of the bottom of the fluid holding tube 106 can be set to have an inverted cone shape. At this time, the shape of the support recess 93a matches the outer contour of the bottom of the fluid holding tube 106. When the bottom of the fluid holding tube 106 is supported on the supporting recess 93a, it can be ensured that the fluid holding tube 106 always maintains an upright state.

In some embodiments, the present invention provides a fluid holding tube adapter, which is characterized in that it comprises an adapter body and a fluid holding tube placement position arranged on the adapter body, and the adapter body is arranged to be installed in the sample processing unit or the functional station on or inside the analytical instrument, the position of the fluid holding tube is set to accommodate the fluid holding tube placement position with an outer diameter of 3 mm to 8 mm and a length of 20 mm to 30 mm, and the fluid holding tube is the relative positions of the functional stations are fixed by the fluid holding tube adapter. In some embodiments, the adapter body is configured to be detachably installed in the functional station. In some embodiments, the adapter body is configured to be fixedly installed in the functional station.

In some embodiments, the fluid holding tube is detachably inserted into the fluid holding tube placement position. In some embodiments, the adapter body outer peripheral portion at least partially conforms to the inner peripheral wall of the functional station. In some embodiments, the cartridge tube outer periphery at least partially abuts the adapter body inner periphery and/or the fluid holding tube placement position inner periphery.

In some embodiments, the sample processing or analysis instrument includes a centrifuge, a spectrometer, and other instruments.

In some embodiments, the fluid holding tube placement position comprises a fluid holding tube insertion hole. In some embodiments, the fluid holding tube placement position further comprises a elastic component configured to: under the condition that a fluid holding tube is inserted into the fluid holding tube insertion hole, the elastic force can act on the fluid holding tube, so that the fluid holding tube can elastically move along the direction of the central axis of the hole of the fluid holding tube insertion hole.

In some embodiments, the fluid holding tube placement position further comprises an outer sleeve disposed along an inner peripheral wall of the fluid holding tube insertion hole, and the elastic component is located in a sleeve inner cavity of the outer sleeve.

In some embodiments, the fluid holding tube placement position further comprises an outer sleeve disposed along an inner peripheral wall of the fluid holding tube insertion hole, and under the condition that the fluid holding tube is inserted into the fluid holding tube insertion hole, the outer sleeve can elastically act on the fluid holding tube, so that the fluid holding tube can elastically move along the direction of the central hole axis of the fluid holding tube insertion hole.

In some embodiments, the invention provides a combination comprising a fluid holding tube and any fluid holding tube adapter provided according to the invention.

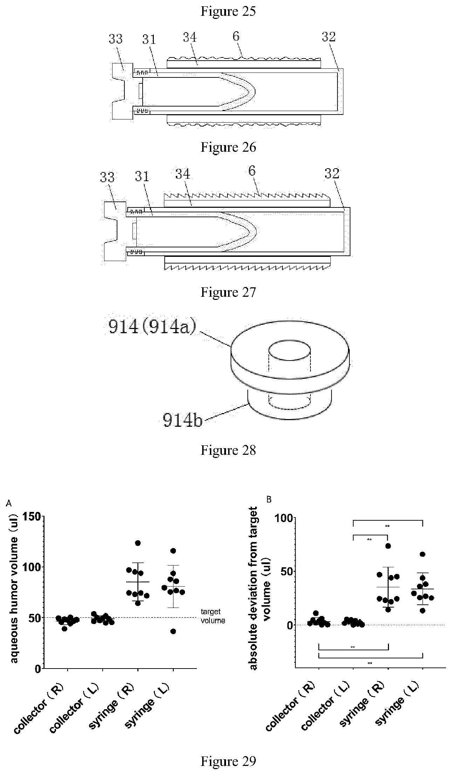

In one embodiment, referring to FIG. 28, the reagent holder 9 further comprises a switching jack 914 removably insertable into the fluid holding tube insertion hole 91, the switching jack 914 comprising a supporting sleeve part 914a and an extending sleeve part 914b connected in an up-down manner. The sleeve cavity of the supporting sleeve part 914a and the sleeve cavity of the extending sleeve part 914b are vertically communicated and jointly form a conversion insertion cavity for detachably inserting the fluid holding tube 106. By selecting the switching jack 914 with different sizes, the fluid holding tube 106 can be always fixed in the fluid holding tube insertion hole 91 with different sizes in a matching manner, so that the difficulty in stably installing the fluid holding tube 106 is avoided. Further, the elastic component may be disposed in the conversion insertion cavity of the switching jack 914, so that the depth of the fluid drawing device extending into the cavity of the fluid holding tube 106 is greatly increased, thereby effectively increasing the sample extraction amount.

The functional stations may include a sample station 95, a reagent station 98, a light measuring station 99, a reaction station 910, a reserved station 912, etc., and may be set according to actual detection requirements.

Furthermore, in some embodiments, the present invention provides a testing apparatus assembly comprising a testing apparatus 10, a fluid holding tube 106, and the reagent holder 9 described above. The fluid holding tube 106 is detachably inserted into the fluid holding tube insertion hole 91 and can be elastically moved along the central axis of the fluid holding tube insertion hole 91 by the elastic force of the elastic component. In the case that the reagent holder 9 is provided with the functional bit outer sleeve 92, the fluid holding tube 106 can pass through the fluid holding tube insertion hole 91 to be inserted into the sleeve inner cavity of the functional bit outer sleeve 92, and preferably, the top end of the fluid holding tube 106 is not higher than the orifice of the fluid holding tube insertion hole 91 at the moment, and the outer diameter of the orifice of the fluid holding tube 106 is the same as the inner diameter of the sleeve inner cavity, so as to prevent the fluid holding tube 106 from shaking and improve the installation stability thereof. Furthermore, the testing apparatus 10 comprises a liquid drawing device arranged for drawing into the lumen of the liquid holding tube 106, for example the liquid drawing device may be provided with a liquid drawing needle 104 for drawing into the lumen of the liquid holding tube 106.

Generally, in order to avoid damage to liquid drawing needle 104 due to a rigid collision, testing apparatus 10 may be provided with a striker protection function that can cause testing apparatus 10 to malfunction and stop operating when liquid drawing needle 104 is subjected to a rigid collision. And under the condition that fluid holding tube 106 receives elastic component's elastic action, when liquid drawing needle 104 touches the diapire that fluid holding tube 106, can turn into the elastic collision with the rigidity collision to avoid getting liquid in-process trigger firing pin protect function, consequently liquid drawing needle 104 can stretch into the liquid of darker position, thereby increase the extraction volume.

In order to ensure that the liquid can be smoothly taken when the liquid drawing needle 104 contacts the bottom wall of the fluid holding tube 106, the liquid drawing needle 104 can be redesigned because the outer end face of the liquid drawing needle 104 provided with the fluid drawing port is a plane end face, the bottom wall of the fluid holding tube 106 is usually a plane wall, and when the plane end face contacts the plane wall, the plane end face and the plane wall are easily in close contact to seal the fluid drawing port, so that the liquid drawing needle 104 cannot draw liquid.

Therefore, in one embodiment, to ensure smooth liquid extraction from liquid drawing needle 104, the outer end surface of liquid drawing needle 104 may be formed as inclined end surface 104a and liquid extraction port may be formed on inclined end surface 104a. With this structure, the liquid inlet is not easily sealed even if the bottom wall of the fluid holding tube 106 is formed in a different shape such as a flat wall. Therefore, the testing apparatus assembly is beneficial to increasing the sample extraction amount during liquid drawing, and can ensure smooth liquid drawing in the fluid drawing process.

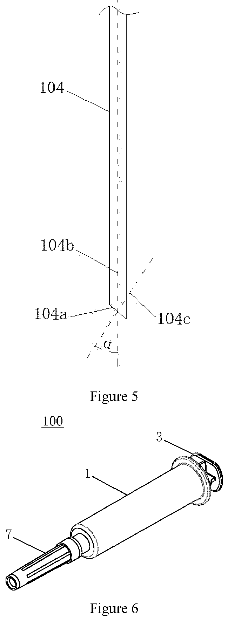

In one embodiment, the liquid drawing needle 104 extends along a straight line and includes a body section and a needle section connected to each other, the outer end of the needle section is formed with a liquid drawing port, and since the outer end surface of the needle section is an inclined end surface, referring to fig. 5, a needle body section central axis 104b of the needle section intersects a liquid drawing port central axis 104c of the fluid drawing port. Specifically, the acute included angle formed by intersection of the needle body section central axis 104b and the fluid drawing port central axis 104c is α, and α can satisfy: 0<α≤0.1°.

It should be noted that the inclined end face 104a may be a curved surface or a flat surface, or the inner bottom wall of the lumen of the fluid holding tube 106 may be a flat surface or a curved surface, as long as the liquid drawing needle 104 is ensured to be in a non-sealing state when contacting the bottom wall of the fluid holding tube 106.

The detection process of the testing apparatus assembly of the present invention is described below by taking a chemiluminescent immunoassay analyzer for aqueous humor sample detection as an example, but it should be understood that other types or fields of testing apparatuses may be disposed in the testing apparatus assembly of the present invention, and the present invention is not limited to the chemiluminescent immunoassay analyzer.

When the testing apparatus is a Chemiluminescence immunoassay analyzer, the reagent holder 9 is in a strip shape and can be provided with a left anchoring piece 94, a sample station 95, a dilution station 96, a washing solution station 97, a reagent station 98, a light measuring station 99, a reaction station 910, a substrate station 911, a reserved station 912 and a right anchoring piece 913 which are sequentially arranged from left to right. Of course, the arrangement order of the functional bits can be set separately according to the actual situation.

Wherein the sample station is used for placing a fluid collection tube, the reagent station is used for pre-loading a reagent, the light measuring station is used for detecting optical signals, the reaction station is used for reacting each component, and the reserved station is used for expanding and reserving for other types of tests. The Chemiluminescence immunoassay analyzer comprises a shell 101, a reaction cabin 102, a hatch 103, the liquid drawing needle 104 and a reagent holder position 105.

When the aqueous humor sample is detected, the hatch 103 of the reaction cabin 102 is opened, the reagent holder 9 inserted with the fluid holding tube 106 is placed in the reagent holder position 105, and the hatch 103 is closed. Then, the detection is started, the Chemiluminescence immunoassay analyzer sequentially takes out the aqueous humor sample and the reagent from corresponding positions through the liquid drawing needle 104, adds the aqueous humor sample and the reagent into the reaction station 910 for warm bath, then transfers the aqueous humor sample to the washing solution station 97 for washing, then transfers the aqueous humor sample to the substrate station 911 for catalysis, and finally transfers the aqueous humor sample to the light measuring station 99 for photometry.

In some embodiments, such as shown in fig. 6-21, the present invention provides an aqueous humor collection device comprising an outer cylinder 1, a puncture needle 2, and a fluid collection tube assembly 3. The outer cylinder 1 is provided with a puncture needle mounting end and a fluid collection tube mounting end which are positioned at two axial ends. The puncture needle 2 can be fixedly arranged at the installation end of the puncture needle, and the fixed installation can be fixed connection or detachable connection. When the puncture needle 2 is fixedly arranged at the installation end of the puncture needle, the puncture needle can be provided with the inner end of the puncture needle positioned in the cavity of the outer cylinder and the outer end of the puncture needle positioned outside the cavity of the outer cylinder. The fluid collection tube assembly 3 may include a fluid collection tube 31 inserted into the outer cylinder cavity from the fluid collection tube mounting end and capable of moving along the axial direction and a stopcock 33 disposed in the outer cylinder cavity for sealing the orifice of the fluid collection tube. Alternatively, the stopcock 33 is in direct contact with the inner circumferential wall of the outer cylinder and the fluid collection tube 31 is not in direct contact with the inner circumferential wall of the outer cylinder. Optionally, the stopcock 33 forms a removable contact with the inner peripheral wall of the outer cylinder, for example, a removable contact with a certain contact resistance, so as to control the axial insertion and extraction movement of the fluid collection tube 31 along the cylinder cavity of the outer cylinder and maintain the stability thereof. Preferably, the static friction coefficient between the stopcock 33 and the inner peripheral wall of the outer cylinder is µ1, the static friction coefficient between the stopcock 33 and the nozzle of the fluid collection tube is µ2, and the following conditions are satisfied: µ1 <µ2. Alternatively, the stopcock 33 may be spaced from the inner peripheral wall of the outer cylinder to ensure that the stopcock 33 and the inner peripheral wall of the outer cylinder do not directly contact each other. For example, the fluid collection tube 31 is in direct contact with the inner circumferential wall of the outer cylinder and the stopcock 33 is not in direct contact with the inner circumferential wall of the outer cylinder. Alternatively, the fluid collection tube 31 (e.g. the outer circumference of the tube orifice of the fluid collection tube) may form a removable contact with the inner circumferential wall of the outer cylinder, for example, a removable contact with a certain contact resistance, so as to control the axial insertion and extraction movement of the fluid collection tube along the cylinder cavity of the outer cylinder and maintain the stability thereof. Optionally, stopcock 33 and fluid collection tube 31 are all separated in the inner circumferential wall of outer cylinder to guarantee stopcock 33 and fluid collection tube 31 and the inner circumferential wall of outer cylinder all not direct contact of each other. For example, the aqueous humor collection device may include a supporting tube rack 32 for fixedly mounting the fluid collection tube 31, and the outer circumferential wall of the support tube holder is in removable contact with the inner circumferential wall of the outer cylinder, for example, removable contact with a certain contact resistance, so as to drive and control the axial insertion and extraction movement of the fluid collection tube and the stopcock along the cylinder cavity of the outer cylinder and maintain the stability thereof.

In some embodiments, when performing anterior chamber puncture using the aqueous humor collection device according to the exemplary embodiment of the present invention, the fluid collection tube assembly 3 may be pushed toward the inner end of the puncture needle, so that the inner end of the puncture needle pierces the soft sleeve 8 and the stopcock 33 and then is inserted into the fluid collection tube cavity, so as to drain the aqueous humor in the anterior chamber to the fluid collection tube cavity through the outer end of the puncture needle and the inner end of the puncture needle. In this collection process, because the fluid collection tube mouth of fluid collection tube is sealed by stopcock 33, the fluid collection tube lumen is in negative pressure state all the time, nevertheless along with the liquid volume in the lumen increases gradually, the difference between lumen internal pressure and anterior chamber internal pressure reduces gradually, consequently can make the drainage speed of aqueous humor reduce gradually to avoid appearing the dangerous condition of intraocular pressure shock, improve the security of puncture drainage greatly. Furthermore, optionally, a marker line may be provided on the fluid collection tube 31 to mark the optimal movement distance of the fluid collection tube 31 so that the fingers do not have to exert excessive force to affect the stability of the axial movement of the fluid collection tube and the stopcock along the bore of the outer cylinder when the inner end of the puncture needle pierces the stopcock 33. Optionally, aqueous humor collection device can include limiting protrusion 6, helps the stability of fluid collection tube and stopcock along outer cylinder chamber axial displacement.

In some embodiments, after aqueous humor finishes gathering, can wholly extract fluid collection tube assembly 3 from outer cylinder, then directly place fluid collection tube 31 in specific detection instrument and carry out specific project and detect, compare with prior art, need not make further transfer to the aqueous humor after gathering, consequently can avoid aqueous humor in the fluid collection tube lumen and external environment to contact, guarantee the purity of the aqueous humor of gathering, thereby improve the accuracy and the reliability of follow-up detection, and can avoid because the waste that causes of transferring the aqueous humor.

In some embodiments, because the static friction coefficient µ1 between the stopcock 33 and the inner peripheral wall of the outer cylinder is defined to be smaller than the static friction coefficient µ2 between the stopcock 33 and the nozzle of the fluid collection tube, or the stopcock 33 is arranged at intervals on the inner peripheral wall of the outer cylinder, the stopcock 33 can be ensured to tightly block the nozzle of the fluid collection tube all the time when the fluid collection tube assembly 3 is integrally pulled out from the cylinder cavity of the outer cylinder, so that the purity of the collected aqueous humor can be ensured more reliably.

In some embodiments, when the stopcock 33 and the inner peripheral wall of the outer cylinder contact each other, in order to better avoid the stopcock 33 from falling off, the stopcock 33 and the inner peripheral wall of the outer cylinder are preferably configured to contact through a smooth surface. For example, the radially outer end surface of the stopcock 33 or the outer cylinder inner peripheral wall surface may be set to a smooth surface, or both the radially outer end surface of the stopcock 33 and the outer cylinder inner peripheral wall surface may be set to a smooth surface.

In some embodiments, the aqueous humor collection device comprises an anti-shaking structure for limiting the radial displacement of the fluid collection tube assembly 3 along the cylindrical cavity of the outer cylinder. In some embodiments, the anti-shaking structure facilitates axial movement of the fluid collection tube assembly during plugging movement while reducing or eliminating radial sway. For example, under this structure, can guarantee that fluid collection tube assembly 3 removes the in-process at the stopcock and only follow axial displacement and cannot appear radially rocking, can not only guarantee puncture in-process puncture needle the inner puncture stopcock 33 of puncturing accurately in order to insert to the fluid collection tube lumen, can also guarantee to extract fluid collection tube assembly 3 steadily after aqueous humor gathers finishes to avoid the aqueous humor in the fluid collection tube lumen to leak, thereby further improve the accuracy and the reliability of follow-up detection.

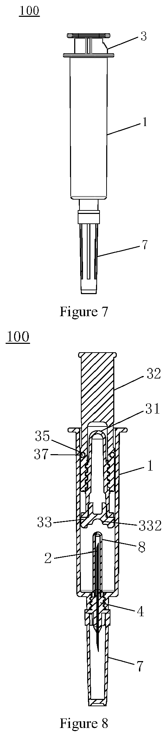

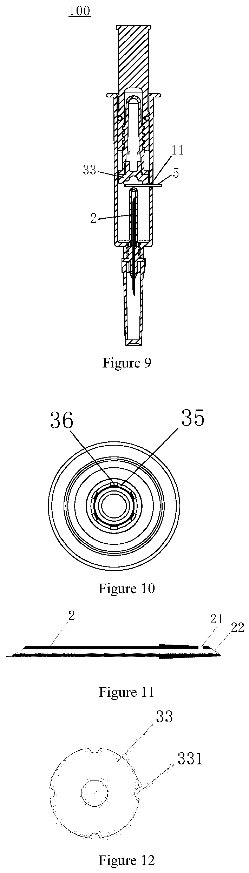

In some embodiments, as shown in fig. 8 and fig. 9, the anti-shaking structure may comprise an annular groove 35 formed on the outer circumferential wall of the fluid collection tube assembly 3 and an annular limiting member 37 provided to the annular groove 35, wherein the radially outer end of the annular limiting member 37 abuts against the inner circumferential wall of the outer cylinder. It should be noted that, the annular limiting member 37 not only can prevent the radial shaking of the fluid collection tube assembly 3 to make the inner end of the puncture needle puncture the stopcock 33 more accurately and prevent deviation during the puncturing process, but also can properly increase the insertion and extraction resistance of the fluid collection tube assembly 3 to make the operator feel a certain pushing hand feeling, thereby avoiding the situation that the puncturing is not accurate due to too fast pushing speed when the fluid collection tube assembly 3 is pushed towards the inner end of the puncture needle, and further avoiding the inner end of the puncture needle from being inserted into the fluid collection tube cavity, which effectively improves the stability and reliability of the puncturing and drainage process. In addition, the liquid collection tube assembly 3 can be prevented from being separated from the outer cylinder when the aqueous humor collection device is taken, thereby improving the reliability of the product. Preferably, a plurality of sets of annular grooves 35 and corresponding annular limiting member 37 may be provided for better results.

In some embodiments, the aqueous humor collection method of the present invention comprises
pushing the rear end of the fluid collection tube assembly to move the fluid collection tube toward the inner end of the needle after the outer end of the needle has penetrated the anterior chamber of the eye, so that the inner end of the needle pierces the soft sleeve 8 and the stopcock 33 and is inserted into the lumen of the fluid collection tube. Alternatively, the soft sleeve originally covering the inner end portion of the puncture needle inserted into the lumen of the fluid collection tube is compressed by the stopcock to generate a resilient force. Optionally, a certain friction force is provided between the compressed soft sleeve which still covers the part of the inner end of the puncture needle which is not inserted into the stopcock and the tube cavity of the fluid collection tube and the inner end of the puncture needle. Optionally, a certain friction force may be provided between the inner end of the puncture needle and the penetrated stopcock, and/or between the annular limiting member and the inner peripheral wall of the outer cylinder.

In some embodiments, after the outer end of the puncture needle is inserted into the anterior chamber of the eye and the inner end of the puncture needle is inserted into the fluid collection tube cavity, aqueous humor in the anterior chamber is drained to the fluid collection tube cavity through the outer end of the puncture needle and the inner end of the puncture needle under the action of the difference between the pressure in the fluid collection tube cavity and the pressure in the anterior chamber. In the aqueous humor collecting process, along with the gradual increase of the liquid amount in the tube cavity, the pressure in the tube cavity and the pressure in the anterior chamber tend to reach the balance, and the aqueous humor is gradually stopped to be drained to the tube cavity of the fluid collection tube through the puncture needle. Optionally, the time from insertion of the inner end of the needle into the lumen of the fluid collection tube until the intraluminal pressure equilibrates with the anterior chamber pressure and/or aqueous humor ceases to drain is at or about 1 s, 2 s, 3 s, 4 s, 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, 30 s or more.

In some embodiments, the aqueous humor collection method of the present invention includes the step of maintaining the insertion of the inner end of the puncture needle into the fluid collection tube lumen for a period of time after the outer end of the puncture needle is inserted into the anterior chamber of the eye and the inner end of the puncture needle is inserted into the fluid collection tube lumen. Optionally, the inner end of the puncture needle is kept inserted into the lumen of the fluid collection tube until the pressure in the lumen and the pressure in the anterior chamber reach equilibrium. Optionally, the inner end of the puncture needle is kept inserted into the lumen of the fluid collection tube until the aqueous humor stops draining. Optionally, maintaining the insertion of the inner end of the puncture needle into the fluid collection tube lumen may include maintaining or applying an external force to the fluid collection tube assembly 3 to prevent the inner end of the puncture needle from popping out of the fluid collection tube lumen. Alternatively, maintaining or applying external force to the fluid collection tube assembly 3 may continue until the intraluminal pressure and anterior chamber pressure equilibrate, as measured from the insertion of the inner end of the puncture needle into the fluid collection tube lumen. Alternatively, maintaining or applying external force to the fluid collection tube assembly 3 may continue until aqueous humor ceases to drain, timed from the insertion of the inner end of the puncture needle into the fluid collection tube lumen. Optionally, the maintaining of the insertion of the inner tip of the puncture needle into the fluid collection tube lumen may last or last about 1 s, 2 s, 3 s, 4 s, 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, 30 s or more from the time the inner tip of the puncture needle is inserted into the fluid collection tube lumen. Alternatively, the external force may be maintained or applied to the fluid collection tube assembly 3 for about 1 s, 2 s, 3 s, 4 s, 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, 30 s or more from the insertion of the inner end of the needle into the fluid collection tube lumen.

In some embodiments, the fluid collection tube assembly may spring back after the outer end of the needle exits the anterior chamber and application of force to the fluid collection tube assembly ceases (e.g., after the index finger is released), thereby allowing the inner end of the needle to naturally spring out of the fluid collection tube lumen and stopcock.

For example, in the whole process of integrally pulling out the fluid collection tube assembly 3 from the outer cylinder cavity, the sum of the friction force between the inner end of the puncture needle and the soft sleeve 8, the friction force between the inner end of the puncture needle and the penetrated stopcock 33 and the friction force between the annular limiting member 37 and the inner peripheral wall of the outer cylinder can be set to be smaller than the resilience force of the soft sleeve 8, so that the inner end of the puncture needle can naturally pop out the fluid collection tube cavity and the stopcock after the outer end of the puncture needle exits from the anterior chamber and the forefinger loosens the fluid collection tube assembly 3, the inner end of the puncture needle does not need to exit from the fluid collection tube cavity and the stopcock by specially pulling out the fluid collection tube assembly 3, and the operation is simplified. In addition, still can set up soft sleeve 8 into spring structure or the soft sleeve that contains spring structure, so can guarantee the stable deformation of soft sleeve 8 at the puncture in-process, can improve the stability of resilience force when soft sleeve 8 kick-backs again, further help adopting the liquid collection tube assembly to remove the in-process along axial displacement and reduce or cannot appear radially rocking at the stopcock.

In some embodiments, in order to reduce the fluid collection difficulty and the fluid collection cost, the annular limiting member 37 may be preferably configured as a readily available and low-cost rubber ring or other elastic ring, and the annular limiting member 37 made of an elastic material is in elastic contact with the inner peripheral wall of the outer cylinder, so as to avoid excessive friction from abrading the inner peripheral wall of the outer cylinder.

In some embodiments, a groove vent hole 36 may be formed on the groove side wall of the annular groove 35 to penetrate along the axial direction of the outer cylinder chamber, so that the cylinder chamber region of the outer cylinder chamber between the stopcock 33 and the puncture needle mounting end can be communicated with the atmosphere through the groove vent hole 36, thereby preventing air embolism from forming in the outer cylinder chamber to affect smooth insertion and extraction of the liquid collection tube assembly 3, and improving the use reliability of the aqueous humor collection device.

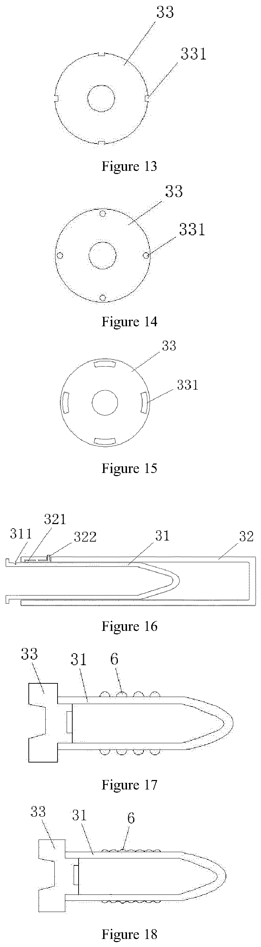

In some embodiments, as shown in fig. 17 to fig. 21, the anti-shaking structure may further include a limiting protrusion 6 protruding from the outer circumferential wall of the fluid collection tube assembly 3 or from the inner circumferential wall of the outer cylinder, and both radial ends of the limiting protrusion 6 are connected to the outer circumferential wall of the fluid collection tube assembly 3 and the inner circumferential wall of the outer cylinder, respectively, so as to prevent the fluid collection tube assembly 3 from shaking radially.

In some embodiments, when the limiting protrusion 6 is provided on the peripheral wall of the fluid collection tube assembly 3, the limiting protrusion 6 may be integrally formed on the peripheral wall of the fluid collection tube 31. Or, the tube kit 34 sleeved on the outer peripheral wall of the fluid collection tube 31 can be arranged in the fluid collection tube assembly 3, and the limiting protrusion 6 is arranged on the outer peripheral wall of the tube kit 34, and the tube kit 34 is a detachable piece, so that the tube kit can be reused, difficulty in forming the limiting protrusion 6 on the tube kit 34 is relatively lower, and production cost reduction is facilitated.

In some embodiments, the tube kit 34 may be provided as a resilient tube kit to prevent the fluid collection tube 31 from breaking due to rigid contact when subjected to a radial stop, thereby reducing the risk of aqueous humor leakage.

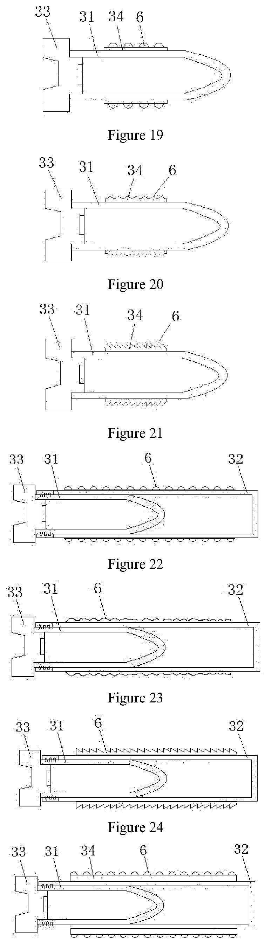

In some embodiments, the anti-shaking structure can include a plurality of limiting protrusion 6 arranged at intervals in sequence along the circumferential direction, and can ensure that the fluid collection tube 31 and the outer cylinder cavity are coaxially arranged, so that the fluid collection tube 31 can be ensured to be smoothly inserted and pulled along the axial direction. In addition, because an exhaust gap is formed between the limiting protrusion 6 which are spaced from each other, the cylinder cavity area of the outer cylinder between the puncture needle mounting end and the stopcock 33 can be communicated with the atmospheric environment through the exhaust gap, thereby avoiding the influence on the smooth plugging and unplugging of the fluid collection tube assembly 3 caused by the formation of an air plug in the cylinder cavity of the outer cylinder. For example, referring to fig. 17 and fig. 19, a plurality of the limiting protrusion 6 arranged at intervals in the circumferential direction may be provided in a convex point shape.

In some embodiments, the limiting protrusion 6 may also be formed to extend continuously in the circumferential direction. When the limiting protrusion 6 is formed on the inner peripheral wall of the outer cylinder, an exhaust gap is preferably formed between the limiting protrusion 6 and the outer peripheral wall of the fluid collection tube assembly 3 to avoid the formation of air lock. When the limiting protrusion 6 is formed on the outer circumferential wall of the fluid collection tube assembly 3, an exhaust gap is preferably formed between the limiting protrusion 6 and the inner circumferential wall of the outer cylinder. For example, referring to fig. 18, fig. 20 and fig. 21, the circumferentially continuously extending limiting protrusions 6 may be provided in a corrugated shape or a helical tooth shape.

In some embodiments, in order to further improve the plugging stability of the fluid collection tube assembly 3, no matter whether the limiting protrusions 6 are sequentially arranged at intervals along the tube length direction of the fluid collection tube assembly 3 or continuously extended and formed along the tube length direction, the total span of the limiting protrusions 6 along the tube length direction is preferably set to be not less than half of the tube length of the fluid collection tube. In other words, when the number of the arrangement of the limiting protrusions 6 in the direction along the length of the tube is sufficiently large, the formation of the fulcrum on the outer circumferential wall of the fluid collection tube assembly 3 can be avoided, thereby more reliably preventing the fluid collection tube assembly 3 from shaking in the radial direction.

In some embodiments, the fluid collection tube assembly 3 further comprises a supporting tube rack 32 for fixedly mounting the fluid collection tube 31. In this structure, the anti-shaking structure can be provided between the outer circumferential wall of the supporting tube rack 32 and the inner circumferential wall of the outer cylinder, as shown in fig. 22 to fig. 27, for example. This supporting tube rack 32 can improve the stability of the fluid collection tube assembly 3 better and can reduce the fluid collection tube assembly 3 owing to receive the collision or press the risk that the lapse breaks. In some embodiments, after aqueous humor is collected, the fluid collection tube needs to be taken out separately, so that the connection mode between the fluid collection tube 31 and the supporting tube rack 32 meets the requirements of stability and quick assembly and disassembly at the same time. Preferably, the fluid collection tube 31 and the supporting tube rack 32 can be fixed by screwing or snapping connection.

For example, referring to fig. 16, the fluid collection tube 31 is connected to the supporting tube rack 32 by a snap fit, and when the fluid collection tube 31 needs to be inserted into the supporting tube rack 32, the fluid collection tube 31 is pushed to the right until the tube rack fastener 321 is snapped into the fluid collection tube clamping groove 311, so that the fluid collection tube 31 and the supporting tube rack 32 can be mutually locked by the snap fit. When the fluid collection tube 31 needs to be taken out, the tube rack button 322 can be pressed, so that the tube rack fastener 321 is lifted to be separated from fluid collection tube clamping groove 311, and the fluid collection tube 31 can be quickly taken out from the supporting tube rack 32.

In some embodiments, the aqueous humor collection device further comprises a needle seat 4 for fixedly mounting the puncture needle 2. Wherein, the outer end of the puncture needle extends out from the outer end of the needle seat, the inner end of the puncture needle extends out from the inner end of the needle seat, and the inner end of the needle seat and the installation end of the puncture needle form quick plug connection, thereby being beneficial to reducing the installation difficulty and improving the assembly efficiency. For example, the inner end of the needle seat and the mounting end of the puncture needle can be in threaded connection or in buckling connection.

In some embodiments, the aqueous humor collection device further comprises an anti-puncture spacing piece 5, wherein the anti-puncture spacing piece 5 is used for isolating the inner end of the puncture needle from the stopcock 33 along the axial direction of the cylindrical cavity of the outer cylinder, so as to prevent the inner end of the puncture needle from puncturing the stopcock 33 in the case of anterior chamber puncture without using the aqueous humor collection device, i.e., to effectively prevent the device from failing before being used. The anti-puncture spacing piece 5 prevents the aqueous humor collection device from being disabled, for example, in the course of transportation or in the case of erroneous operation by medical staff. Of course, in order not to affect the normal puncture drainage of the aqueous humor collection device, the anti-puncture spacing piece 5 is provided as a detachable member, and the anti-puncture spacing piece 5 can be detached before the puncture is performed.

For example, referring to fig. 9, a spacer inserting groove 11 may be provided in the peripheral wall of the outer cylinder 1 such that the spacer insertion groove 11 communicates with the outer cylinder chamber at a chamber region between the inner end of the puncture needle and the stopcock 33, and the anti-puncture spacing piece 5 may be inserted into the chamber region through the spacer inserting groove 11 and placed between the inner end of the puncture needle and the stopcock 33, thereby isolating the inner end of the puncture needle and the stopcock 33 from each other. When the anti-puncture spacing piece 5 needs to be removed, the anti-puncture spacing piece can be quickly pulled out of the spacer insertion groove 11, and the operation is quite convenient.

In some embodiments, the aqueous humor collection device further comprises a detachable limiting structure to prevent the inner end of the puncture needle from puncturing the stopcock to disable the aqueous humor collection device and/or prevent the fluid collection tube assembly 3 from falling off from the outer cylinder in the transportation process due to vibration or mis-operation of medical staff and the like.

Optionally, in a packaged or unused aqueous humor collection device, the limiting structure is located at the rear end of the supporting tube rack. Optionally, in the packaged or unused aqueous humor collection device, the limiting structure is positioned at the installation end of the outer cylinder fluid collection tube. Optionally, in the packaged or unused aqueous humor collection device, at least a part of the limiting structure is located between the installation end of the fluid collection tube of the outer cylinder and the rear end of the supporting tube rack. Optionally, the limiting structure can prevent the supporting tube rack and the outer cylinder from axial relative displacement, so as to prevent the inner end of the puncture needle from puncturing the stopcock 33 in the case of anterior chamber puncture without using the aqueous humor collection device, and/or prevent the fluid collection tube assembly 3 from falling off from the outer cylinder due to gravity or external force. This limiting structure is the detachable piece, before carrying out the puncture, can demolish it.

In some embodiments, as shown in fig. 12 to fig. 15, in order to better avoid forming an air plug in the outer cylinder cavity and thus affecting the smooth insertion and extraction of the fluid collection tube assembly 3, a stopcock exhaust passage 331 arranged along the extending direction of the outer cylinder cavity may be formed at the radial outer edge of the stopcock 33, and the cavity area of the outer cylinder cavity between the stopcock 33 and the puncture needle mounting end may be communicated with the atmosphere through the stopcock exhaust passage 331 and the peripheral wall air exhaust gap between the fluid collection tube 31 and the inner peripheral wall of the outer cylinder. In other words, the outer cylinder chamber communicates in sequence with the chamber region between the stopcock 33 and the puncture needle mounting end, the stopcock exhaust passage 331 and the circumferential wall exhaust gap, and the atmosphere.

In some embodiments, to improve the ventilation effect and make the structure of the stopcock 33 more uniform and reasonable, a plurality of stopcock exhaust passage 331 are provided, which extend along the axial direction of the cylinder cavity of the outer cylinder and are sequentially arranged at intervals along the circumferential direction of the stopcock 33.

The specific shape of the stopcock exhaust passage 331 may be determined according to actual needs. In some embodiments, referring to fig. 12 and fig. 13, a stopcock vent groove provided with a groove opening facing radially outward may be formed on the outer circumferential wall of the stopcock 33 as a stopcock vent passage 331. In other words, the stopcock exhaust passage 331 may be provided as an exhaust path whose circumferential wall is open. Alternatively, referring to fig. 14 and fig. 15, the passage peripheral wall of the stopcock exhaust passage 331 may be provided as a closed peripheral wall, that is, the stopcock exhaust passage 331 may be provided as an exhaust passage whose peripheral wall is closed. In addition, the cross section of the stopcock exhaust passage 331 may be formed in various shapes such as a semicircle, a square, or an arc.

In some embodiments, the stopcock 33 includes a plug central portion for sealing the opening of the fluid collection tube and a stopcock outer ring portion located radially outside the stopcock central portion and provided with the stopcock exhaust passage 331, and in order to ensure the inside and outside ventilation, the stopcock outer ring portion may be at least partially set to be a loose ventilation structure, but it is still necessary to ensure the sealing performance of the stopcock central portion.

In some embodiments, in order to ensure that the inner end of the puncture needle does not shake when puncturing the stopcock 33, thereby affecting the puncturing accuracy, a puncture guide blind hole 332 for guiding the inner end of the puncture needle to penetrate can be arranged at the end of the stopcock 33, and the outer contour shape of the inner end of the puncture needle is matched with the shape of the puncture guide blind hole 332. For example, referring to fig. 8, the inner end of the needle and the puncture guide blind hole 332 may be configured in matching long conical shapes.

It should be noted that, during the process of puncturing the stopcock 33 by the inner end of the puncture needle, the axial water outlet hole 22 at the inner end of the puncture needle may be blocked by the stopcock material, so that the drainage is blocked and the device cannot work normally. Therefore, in some embodiments, in order to avoid the occurrence of the flow breaking, a peripheral wall water outlet hole 21 communicating with the needle chamber of the puncture needle 2 may be provided on the peripheral wall of the inner end of the puncture needle, and in this structure, even if the axial water outlet hole 22 is blocked by the stopcock material, the aqueous humor can flow out into the fluid collection tube cavity through the peripheral wall water outlet hole 21, thereby ensuring the operational reliability of the device.

In addition, the axial distance between the peripheral wall water outlet hole 21 and the axial water outlet hole 22 should not be too large, otherwise, after the axial water outlet hole 22 is inserted into the tube cavity of the fluid collection tube, the peripheral wall water outlet hole 21 is still located in the stopcock 33 and is blocked, so that the peripheral wall water outlet hole 21 is disabled. For this reason, in some embodiments, the axial distance between the hole center of the peripheral wall water outlet hole 21 and the hole center of the axial water outlet hole 22 may preferably be set to be not more than 3 mm, so as to ensure that the peripheral wall water outlet hole 21 can be located in the fluid collection tube cavity without being blocked after the axial water outlet hole 22 is inserted into the fluid collection tube cavity.

Next, optional parameter settings of some components and the like in the aqueous humor collection device of the present exemplary embodiment will be described.

In some embodiments, the puncture needle 2 may have an outer diameter of 0.31 mm (30G) to 0.91 mm (20G) and an inner diameter that is adjustable according to the outer diameter. Alternatively, the puncture needle 2 may be provided with an outer diameter of 0.5mm (25G) and an inner diameter of at least about 0.232 mm. Optionally, the outer diameter of the needle 2 is at or about 0.3 mm, 0.4 mm, 0.5mm, 0.6 mm, 0.7 mm, 0.8 mm, or 0.9 mm. Optionally, the puncture needle 2 has an inner diameter of 0.15 mm to 0.45 mm, for example, the puncture needle 2 has an inner diameter of or about 0.1 mm, 0.2 mm, 0.3 mm, or 0.4 mm.

In some embodiments, the total length of the puncture needle 2 can be set from 20mm to 65 mm, such as where the total length of the puncture needle 2 is at or about 25mm, 30mm, 35mm, 40mm, 45 mm, 50mm, 55 mm, or 60 mm. Alternatively, the projecting length of the puncture needle 2 from the needle seat 4 may be set to 1.5mm to 15mm, for example, 1.5mm to 5mm, 5mm to 7.5mm, 7.5mm to 10mm, 10mm to 12.5mm or 12.5mm to 15mm, preferably 6 mm. Alternatively, the projecting length of the puncture needle 2 projecting inwardly from the needle seat 4 may be set to 10mm to 30mm, for example, 10mm to 15mm, 15mm to 20mm, 20mm to 25mm, or 25mm to 30mm, preferably 15 mm. Alternatively, the length of the portion of the puncture needle 2 located in the needle seat 4 may be set to 10mm to 20mm, for example 10mm to 15mm, or 15mm to 20mm, preferably 14mm, in view of the stability of the mold. Alternatively, for example, the puncture needle 2 may have an overall length of at or about 35mm, an outward extension from the needle seat 4 of at or about 6mm, an inward extension from the needle seat 4 of at or about 15mm, and a portion within the needle seat 4 of at or about 14 mm.

The needle tip bevel of the puncture needle 2 can be set to be 5° to 50°, for example, 8°, 10°, 12°, 15°, 17°, 19°, 43°, 45°, 47°.

In some embodiments, the volume of aqueous humor collected by the puncture needle 2 may be 10 µL to 100 µL, and may be set to, for example, 32µL, 37.5 µL, 40 µL, 45 µL, 48 µL, 50 µL, 51 µL, 55 µL, 60 µL, 62.5 µL, 69 µL, and the like.

In some embodiments, the target volume of aqueous humor collected by the puncture needle 2 may be 10µL to 100 µL, and may be set, for example, to 32 µL, 37.5 µL, 40 µL, 45 µL, 48 µL, 50 µL, 51 µL, 55 µL, 60 µL, 62.5 µL, 69 µL, and the like. In some embodiments, the accuracy of aqueous humor collection is facilitated using the aqueous humor collection devices and methods of the present invention, for example, the volume of aqueous humor collected can reach a target volume of ± 1%, a target volume of + 5%, a target volume of + 10%, a target volume of + 15%, a target volume of + 20%, or a target volume of + 25%.

In some embodiments, the fluid collection tube 31 may be contoured to accommodate a centrifuge with a maximum centrifugal force of 4000 gₙ.

In some embodiments, the volume of the fluid collection tube 31 (i.e., the fluid collection tube 31 under negative pressure) provided with the stopcock 33 may be set to 8 µL to 310 µL (e.g., may be set to 10 µL, 50 µL, 100 µL, 150 µL, 200 µL, 250 µL, 300 µL, etc.), and the gas pressure range thereof may be set to 1 × 10³ Pa to 1 × 10⁴ Pa. Alternatively, the pressure range in the fluid collection tube 31 provided with the stopcock 33 (i.e., the fluid collection tube 31 of negative pressure) is set to be between 8mmHg and 40mmHg, for example, 8mmHg to 10mmHg, 10mmHg to 15mmHg, 15mmHg to 20mmHg, 20mmHg to 25mmHg, 25mmHg to 30mmHg, 30mmHg to 35mmHg, or 35mmHg to 40 mmHg.

In some embodiments, the inner diameter of the fluid collection tube 31 may be set to 2.5mm to 6mm, the outer diameter may be set to 3mm to 8mm, and the tube length may be set to 20mm to 30 mm. The outer diameter of the outer cylinder 1 may be set to 6mm to 16mm, the length of the supporting tube rack 32 may be set to 20mm to 50mm and the diameter thereof may be set to 5mm to 20mm when the supporting tube rack 32 is a circular tube rack.

In addition, the outer cylinder 1 can be made of 304 stainless steel, the needle seat 4, the supporting tube rack 32 and the soft sleeve 8 can be made of PP materials, the fluid collection tube 31 can be made of PET materials, and the stopcock 33 and the annular limiting member 37 can be made of rubber.

In some embodiments, the puncture needle 2 has an overall length of at or about 35mm, an outward extension from the needle seat 4 of at or about 6mm, an inward extension from the needle seat 4 of at or about 15mm, and a portion within the needle seat 4 of at or about 14 mm; the outer diameter of the puncture needle 2 is set to be 0.5mm (25G); the oblique angle of the needle tip of the puncture needle 2 is set to be 17° ± 2°; the volume of the fluid collection tube 31 provided with the stopcock 33 is set to be 8 µL to 310 µL, and the air pressure range in the fluid collection tube is set to be 8 mmHg to 40 mmHg.

In some embodiments, the present invention provides a method of collecting aqueous humor from a cornea, comprising:
the outer end of the puncture needle 2 of the aqueous humor collection device is punctured into the anterior chamber of the eye;
pushing the rear end of the fluid collection tube assembly 3 to move the fluid collection tube 31 in the fluid collection tube assembly 3 towards the inner end of the puncture needle 2, so that the inner end of the puncture needle passes through the stopcock 33 and enters the fluid collection tube 31;
the aqueous humor of the anterior chamber is caused to pass through the puncture needle 2 and flow into the fluid collection tube 31 under a pressure difference between the intraocular pressure and the negative pressure;
withdrawing the aqueous humor collection device from the anterior chamber; and the fluid collection tube assembly 3 is taken out from the outer cylinder 1.

In some embodiments, the step of pushing the rear end of the fluid collection tube assembly 3 may comprise:
the aqueous humor collection device is held with one hand and the index finger pushes the rear end of the fluid collection tube assembly 3.

In some embodiments, aqueous humor from the anterior chamber passes through the puncture needle 2 and flows into the fluid collection tube 31 at a pressure differential between the intraocular pressure and the negative pressure in the fluid collection tube 31 for a duration of about 1 s, 2 s, 3 s, 4 s, 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, 30 s, or more.

In some embodiments, the step of withdrawing the fluid collection tube assembly 3 from the outer cylinder 1 may comprise: external force is not applied to the fluid collection tube assembly, or the external force applied to the fluid collection tube assembly is stopped or reduced, and the fluid collection tube assembly is allowed to rebound. Alternatively, the fluid collection tube assembly 3 can be ejected naturally without the need to pull the riser package out exclusively by applying an external force.

In some embodiments, the present invention provides a method for collecting aqueous humor from an eye of a subject, comprising:
the outer end of the puncture needle 2 of the aqueous humor collection device is punctured into the anterior chamber of the eye, wherein the pressure in the fluid collection tube 31 is lower than the intraocular pressure of a subject;
pushing the rear end of the fluid collection tube assembly 3 to move the fluid collection tube 31 in the fluid collection tube assembly 3 towards the inner end of the puncture needle 2, so that the inner end of the puncture needle passes through the stopcock 33 and enters the fluid collection tube 31; allowing aqueous humor in the anterior chamber of the eye to pass through the puncture needle 2 and into the fluid collection tube 31 at a pressure differential between the intraocular pressure and the negative pressure;
withdrawing the aqueous humor collection device from the anterior chamber; and the fluid collection tube assembly 3 is taken out from the outer cylinder 1.

In some embodiments, the step of pushing the rear end of the fluid collection tube assembly 3 may comprise:
the aqueous humor collection device is held with one hand and the index finger pushes the rear end of the fluid collection tube assembly 3.

In some embodiments, aqueous humor from the anterior chamber passes through the puncture needle 2 and flows into the fluid collection tube 31 at a pressure differential between the intraocular pressure and the negative pressure in the fluid collection tube 31 for a duration of about 1 s, 2 s, 3 s, 4 s, 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, 30 s, or more.

In some embodiments, the step of withdrawing the fluid collection tube assembly 3 from the outer cylinder 1 may comprise: external force is not applied to the fluid collection tube assembly, or the external force applied to the fluid collection tube assembly is stopped or reduced, and the fluid collection tube assembly is allowed to rebound. Alternatively, the fluid collection tube assembly 3 can be ejected naturally without the need to pull the riser package out exclusively by applying an external force.

In some embodiments, the present invention provides a method for collecting aqueous humor from an eye of a subject, comprising:
the outer end of the puncture needle 2 of the aqueous humor collection device is punctured into the anterior chamber of an eye, wherein the pressure in the fluid collection tube 31 is between 8 mmHg and 40mmHg and is lower than the intraocular pressure of a subject;
holding the aqueous humor collection device with one hand, and pushing the rear end of the fluid collection tube assembly 3 with the index finger to move the fluid collection tube 31 in the fluid collection tube assembly 3 towards the inner end of the puncture needle 2 so that the inner end of the puncture needle passes through the stopcock 33 and enters the fluid collection tube 31;
allowing aqueous humor of the anterior chamber of the eye to pass through the puncture needle 2 and flow into the fluid collection tube 31 at a pressure differential between the intraocular pressure and the negative pressure, which may continue or last for about 1 s, 2 s, 3 s, 4 s, 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, or 30 s;
withdrawing the aqueous humor collection device from the anterior chamber;
stopping or reducing the application of external force to the fluid collection tube assembly and allowing the fluid collection tube assembly to rebound; and
the fluid collection tube assembly 3 is taken out from the outer cylinder 1.

In some embodiments, the method may further include selecting a negative pressure inside the fluid collection tube 31 that is below the intraocular pressure of the subject's eye. Optionally, the subject's intraocular pressure is between 7mmHg and 50mmHg, e.g., 7mmHg to 10mmHg, 10mmHg to 20mmHg, 20mmHg to 30mmHg, 30mmHg to 40mmHg, or 40mmHg to 50 mmHg. Alternatively, the pressure range in the fluid collection tube 31 provided with the stopcock 33 (i.e., the fluid collection tube 31 of negative pressure) may be set to be between 8mmHg and 40mmHg, for example, 8mmHg to 10mmHg, 10mmHg to 15mmHg, 15mmHg to 20mmHg, 20mmHg to 25mmHg, 25mmHg to 30mmHg, 30mmHg to 35mmHg, or 35mmHg to 40 mmHg. Optionally, the pressure range in the fluid collection tube 31 provided with the stopcock 33 (i.e., the negative pressure collection tube 31) is about 0.5 mmHg, 1 mmHg, 2 mmHg, 3 mmHg, 4 mmHg, 5mmHg, 6 mmHg, 7mmHg, 8mmHg, 9 mmHg, or 10mmHg lower than the intraocular pressure of the subject.

In some embodiments, the invention provides a method of assembling the above-described aqueous humor collection device. Optionally, the assembly method comprises assembling the stopcock 33 with the fluid collection tube 31 in a sub-pressure tank to provide sub-pressure within the fluid collection tube lumen. Alternatively, the negative pressure in the sub-pressure tank can be adjusted to fit fluid collection tubes of different negative pressure values. For example, the aqueous humor collection device can be set to be different in model and correspond to different negative pressure values of the fluid collection tube. According to the intraocular pressure of the subject, a proper negative pressure value of the fluid collection tube can be selected to collect the aqueous humor safely and reliably. The animal experiment process using the aqueous humor collection device of the present invention will be shown below to more intuitively embody the technical effects of the aqueous humor collection device.

### 1. Purpose of the test

The device for collecting the aqueous humor has the advantages of safety, accuracy, convenience, high operation efficiency and the like.

### 2. Test animals and groups

In the animal test, the New Zealand white rabbits are taken as test animals, and 20 New Zealand white rabbits with the body weight of 2.1 kg to 2.6 kg and no abnormal eyes are brought in. In addition, a commonly used 25G 1ml tuberculin syringe (manufacturer's specification is shown in Table 1, hereinafter simply referred to as "1 ml syringe") was used as a comparative example.

In the experiment, rabbits were randomly divided into group 1 and group 2 at the ratio of 1:1, each of which contained 10 individuals. In consideration of the influence of the left-right-hand operation on the anterior chamber puncture, in group 1, the right-hand puncturing operation was performed by collecting the aqueous humor of the right eye with an aqueous humor collection device and collecting the aqueous humor of the left eye with a 1ml syringe. In group 2, the anterior aqueous humor of the left eye was collected with an aqueous humor collection device, and the anterior aqueous humor of the right eye was collected with a 1ml syringe, all of which were subjected to a puncturing operation with the left hand.

In addition, all procedures in this trial were performed by the same experimental operator with extensive intracerebral puncture and aqueous collection experience.

### 3. Product information

**TABLE 1**

| Group | Name of product | Product information | Storage conditions |
|---|---|---|---|
| Examples | Disposable anterior chamber puncture device (i.e. aqueous humor collection device of the present invention) | Batch number: DAHC1908001 specification: SnovoDAHC 1-50 manufacturer: Hangzhou Huashinuowei medical science and technology Co Ltd | Stored at normal temperature, and has relative humidity not more than 80% |
| Comparative example | Disposable tuberculin syringes (i.e. 1ml syringe) | Batch number: 8264079 specification: 1ML LS 25GA 5/8 IN vendor: becton Dickinson Medical (S) Pte. Ltd. | Storage at normal temperature |

### 4. Puncture procedure and post-operative assessment

Rabbits were anesthetized by intramuscular injection of xylazine hydrochloride injection (lomannin) and laid on their side during the puncture. Local anesthesia was performed 5 minutes before the operation, and oxybuprocaine hydrochloride eye drops (binomi) were dropped 1 time in both eyes. In the puncture process, the rabbit is in a lateral position, a needle is inserted from the cornea which is about 1 mm to 2 mm away from the corneal limbus, the needle head is kept parallel to the plane of the iris, and attention needs to be paid to avoid the anterior lens capsule.

Wherein, the aqueous humor collection device performs anterior chamber puncture and anterior chamber aqueous humor collection with one hand according to the aqueous humor collection method. 1 ml syringe was then punctured into the anterior chamber with one hand holding the syringe and slowly pull the needle stopcock with the other hand to gradually draw the aqueous humor into the syringe, and then transfer the aqueous humor to the micro centrifuge tube.

To increase the comparability of these two procedures, the target draw volumes for the 1ml syringe set and aqueous humor collection device were set to be identical and both set to 50 µL. A stopwatch was used to record the time taken from the beginning of the puncture to the completion of the whole procedure for anterior aqueous humor collection during the experiment.

The measurement and calculation method for the volume of the collected aqueous humor of each group is as follows:
before the puncturing operation, the net weight m1 (mg) of the fluid collection tube 31 (internal part of the aqueous humor collection device) or the micro centrifuge tube (corresponding to the method of drawing aqueous humor with a 1mL syringe) was weighed by a precision balance, and the weight m2 (mg) of the fluid collection tube 31 or the micro centrifuge tube containing aqueous humor was measured after puncturing, and the collected weight m = m2-m1 (mg) of the aqueous humor. The net weight m (mg) of the aqueous humor was divided by its approximate density of 1.0mg/µL to obtain the volume (µL) of collected aqueous humor.

Immediately after surgery, the anterior chamber was evaluated under a slit lamp and the leakage of aqueous humor at the puncture station was detected using the Seidel test. The anterior segment was examined again with slit lamps 7 days after the operation.

### 5. Statistical method

Statistical analysis used SPSS version 22.0 (IBM corporation, USA). The T-test and paired T-test are used to compare independent variables and paired variables, respectively. P < 0.05 was considered statistically different.

### 6. Test results

### 6.1 general evaluation

Of the 20 New Zealand white rabbits, one rabbit from group 1 was accidentally punctured and one rabbit from group 2 died under anesthesia, so that only 18 rabbits (36 eyes) were observed and counted after surgery. The behavioral activities and food intake of 18 rabbits were normal in 7 days after surgery, the mental state was still clear, and no obvious adverse reactions such as congestion of eyes and increased secretion were observed.

### 6.2 evaluation of safety and efficacy

The 36 eyes are divided into the following four subgroups according to the adopted instruments and different operators during puncture: group CR: aqueous humor collection device-right hand operating group; CL group: aqueous humor collection device-left-handed operation group; SR set: 1ml syringe-right hand operating group; SL group: 1ml syringe-left hand operated group. Safety and efficacy between subgroups are evaluated below in terms of aqueous humor collection, operating time, anterior segment examination, incision closure, etc.

### Volume of anterior aqueous humor collected (aqueous humor volume)

**TABLE 2 anterior aqueous humor Collection for each group**

| Group | Range (µL) | Mean.± standard deviation (.µL) |
|---|---|---|
| CR | 39.20-50.40 | 46.66 ± 3.37 |
| CL | 45.00-53.60 | 48.71 ± 2.88 |
| SR | 64.00-123.50 | 85.11 ± 18.7 |
| SL | 36.80-115.80 | 80.68 ± 20.87 |

The collected anterior aqueous humor volumes for each set are shown in table 2 and figure 29. Wherein CR or collector (R) represents an aqueous humor collection device-right hand operating group, CL or collector (L) represents an aqueous humor collection device-left hand operating group, SR or syringe (R) represents a 1ml syringe-right hand operating group, and SL or syringe (L) represents a 1ml syringe-left hand operating group.

The absolute value of the target collection (50µL) bias (ADTV) for the CR and CL groups were 3.43 ±3.27 (range 0.40 to 10.80) µL and 2.51 ±1.75 (range 0.20 to 5.00) µL, respectively.

The absolute value of target collection volume (50µL) bias (ADTV) for SR and SL groups was between 35.11 ± 18.70 µL (range 14.00 to 73.50) µL and 33.61 ± 14.95 (range 13.20 to 65.80) µL, respectively.

ADTV was significantly lower for both aqueous humor collection device subsets (CR and CL groups) than for both 1ml syringe subsets (SR and SL groups). However, there were no statistical differences between the two subgroups of aqueous humor collection devices and between the two subgroups of 1ml syringes (P > 0.05).

### 6.2.2 operation time

**TABLE 3 operation time for various subgroups**

| Group | Range(s) | Mean. ± standard deviation(s) |
|---|---|---|
| CR | 10.89-17.67 | 14.41 ±2.06 |
| CL | 12.45-18.83 | 15.53 ±2.13 |
| SR | 17.23-23.57 | 20.16 ±2.46 |
| SL | 17.23-25.95 | 20.11 ±2.79 |

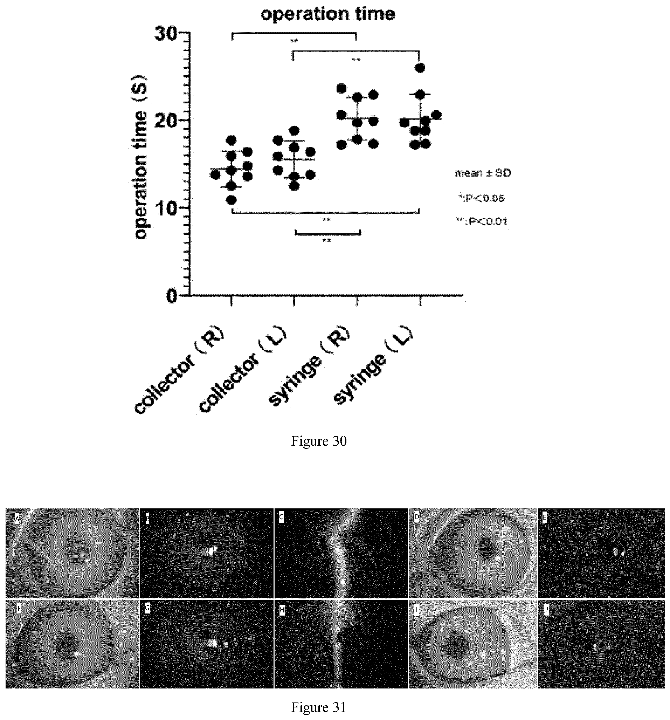

The operating time for each subgroup is shown in table 3 and fig. 30. Wherein CR or collector (R) represents an aqueous humor collection device-right hand operating group, CL or collector (L) represents an aqueous humor collection device-left hand operating group, SR or syringe (r) represents a 1ml syringe-right hand operating group, and SL or syringe (L) represents a 1ml syringe-left hand operating group.

As shown in FIG. 30, the two subgroups using aqueous humor collection devices were operating at shorter times than the two subgroups of 1ml syringes, with statistical significance of the difference (P < 0.01), but no statistical difference between the left-and right-handed operating subgroups (P > 0.05). Different degrees of iris hyperemia and glary flashes were seen immediately after paracentesis, but were essentially resolved one week post-surgery (see figure 31). The Seidel test performed immediately after puncture showed good puncture closure and no leakage of aqueous humor from the anterior aqueous humor of all tested eyes (see fig. 31).

Referring to FIG. 31, the rabbit was examined immediately after the anterior chamber puncture for both iris hyperemia (A, F) and glary flashes (B, G). Seidel tests showed no anterior aqueous humor leakage at the puncture (C, H). Both iris congestion (D, I) and anterior chamber flare (E, J) resolved completely one week after puncture. Note that the rabbit's left eye was drawing aqueous humor (A to E) with a 1ml syringe, while the right eye was drawing aqueous humor (F to J) from an aqueous humor collection device.

### 7. Conclusion

The single use anterior aqueous humor penetrator (i.e., the aqueous humor collection device of the present invention) employed in this test has a number of substantial advantages over previous syringe and pipette based techniques.

First, a complete anterior aqueous humor puncture and aqueous anterior aqueous humor sampling procedure can be easily accomplished with a single hand through a series of simple manipulations. Second, accurate sampling of anterior aqueous humor is achieved for the first time. Third, the operator does not need to pay attention to the volume of the specimen collected during sampling, nor to carefully control the pressure applied to the pipette or the force pulling the pin, but instead can focus all of the attention on the eye being operated. Fourth, the other hand may help stabilize the patient's head or aid in fixing the eye during operation. These designs greatly improve the safety of aqueous humor collection, reduce the dependence on patient compliance, and allow for operation in an outpatient setting with or without the aid of slit lamps. The fifth advantage is that no specimen transfer is required after collection of the anterior aqueous humor because the fluid collection tube 31 of the aqueous humor collection device is itself a micro centrifuge tube. The function also helps to avoid specimen loss in the transferring process, improves the operation efficiency and avoids specimen pollution.

No aqueous leakage and anterior aqueous humor collapse was observed in all eyes tested. As in the previous study, different degrees of iris hyperemia and aqueous flash were observed in all four subgroups after paracentesis, but resolved at 1 week. The above results indicate that both the single use intracerebral puncture instrument and the conventional syringe sampling method are very safe.

In conclusion, the animal experiment proves that the novel disposable anterior aqueous humor puncture outfit realizes the one-hand operation and the accurate quantitative collection of anterior aqueous humor, and has higher safety and accuracy compared with the traditional anterior aqueous humor sampling method using a syringe.

In an embodiment of the present application, there is provided an aqueous humor collection device including:
the outer cylinder is provided with a puncture needle mounting end and a fluid collection tube mounting end which are positioned at two axial ends;
the puncture needle is fixedly arranged at the installation end of the puncture needle and is provided with an inner end of the puncture needle positioned in the cylinder cavity of the outer cylinder and an outer end of the puncture needle positioned outside the cylinder cavity of the outer cylinder;
the fluid collection tube assembly comprises a fluid collection tube which is inserted into the outer cylinder cavity from the fluid collection tube mounting end and can be inserted and pulled in the axial direction, and a stopcock which is arranged in the outer cylinder cavity and is used for sealing the tube orifice of the fluid collection tube;
wherein, the coefficient of static friction between stopcock and the interior peripheral of outer cylinder is µ1, and the coefficient of static friction between stopcock and the fluid collection tube mouth of tube is µ2, and satisfies: µ1 <µ2;
or the stopcock is arranged at intervals on the inner peripheral wall of the outer cylinder.

In the embodiment of the application, smooth contact is formed between the stopcock and the inner peripheral wall of the outer cylinder.

In this application embodiment, aqueous humor collection device is still including being used for spacing fluid collection tube assembly along outer cylinder chamber radial displacement's anti-shaking structure.

The anti-shaking structure comprises an annular groove formed in the outer peripheral wall of the fluid collection tube assembly and an annular limiting member arranged in the annular groove, and the radially outer end of the annular limiting member is abutted to the inner peripheral wall of the outer cylinder.

In the embodiment of the present application, the annular limiting member is a rubber ring.

In the embodiment of the application, the groove side wall of the annular groove is provided with a groove vent hole which is communicated along the axial direction of the outer cylinder cavity, and the cylinder cavity area of the outer cylinder cavity between the stopcock and the puncture needle mounting end is communicated with the groove vent hole.

In the embodiment of the application, the aqueous humor collection device further comprises a needle base for fixedly mounting the puncture needle, and the inner end of the needle base is connected with the mounting end of the puncture needle in a quick plugging manner.

In the embodiment of the application, the inner end of the needle seat and the mounting end of the puncture needle form threaded insertion or buckling insertion.

In the embodiment of the application, the aqueous humor collection device also comprises an anti-puncture spacing piece which is used for isolating the inner end of the puncture needle from the stopcock along the axial direction of the tube cavity of the outer cylinder, and the anti-puncture spacing piece is a detachable piece.

In the embodiment of the application, the peripheral wall of the outer cylinder is provided with the isolating piece inserting groove, the cylinder cavity area of the cylinder cavity of the outer cylinder between the inner end of the puncture needle and the stopcock is communicated with the anti-puncture spacing piece, and the anti-puncture spacing piece is inserted into the cylinder cavity area through the spacer inserting groove.

In an embodiment of the present application, there is provided an aqueous humor collection device including:
the outer cylinder comprises a puncture needle mounting end and a fluid collection tube mounting end;
the puncture needle is fixedly arranged at the puncture needle mounting end and comprises a puncture needle inner end and a puncture needle outer end which respectively extend out of two axial sides of the puncture needle mounting end;
the fluid collection tube assembly comprises a fluid collection tube which is inserted into the outer cylinder cavity from the fluid collection tube mounting end and can be inserted and pulled in the axial direction, and a stopcock which is arranged in the outer cylinder cavity and is used for sealing the tube orifice of the fluid collection tube;
wherein, stopcock and the coaxial setting in outer cylinder cavity and the radius of stopcock are less than the radius of inner peripheral wall of the outer cylinder, and the periphery wall of the fluid collection tube assembly is equipped with the annular locating part that annular groove and hoop annular groove set up, and the radially outer end of the annular limiting member abuts against the inner peripheral wall of the outer cylinder.

In the embodiment of the application, the radial distance between the radial outer end of the stopcock and the inner peripheral wall of the outer cylinder is not less than 1 mm.

In this application embodiment, the fluid collection tube assembly is including supporting tube rack, and the fluid collection tube is inserted into the inner cavity of the inner chamber of the supporting tube rack, and the annular groove is arranged on the outer peripheral wall of the supporting tube rack.

In the embodiment of the application, a threaded connection or a buckling connection is formed between the fluid collection tube and the supporting tube rack.

In the embodiment of the application, the groove side wall of the annular groove is formed with a groove vent hole which is communicated along the axial direction of the outer cylinder cavity, and the cylinder cavity of the outer cylinder cavity is communicated with the groove vent hole in the cylinder cavity area between the stopcock and the puncture needle mounting end.

In the embodiment of the present application, the annular limiting member is an elastic component.

In the embodiment of the application, the aqueous humor collection device further comprises a needle seat for fixedly mounting the puncture needle, and the inner end of the needle seat is connected with the mounting end of the puncture needle in a quick plugging manner.

In the embodiment of the application, a threaded insertion or a buckling insertion is formed between the inner end of the needle seat and the mounting end of the puncture needle.

In an embodiment of the application, the aqueous humor collection device further comprises an anti-puncture spacing piece for isolating the inner end of the puncture needle from the stopcock along the axial direction of the cylinder cavity of the outer cylinder, and the anti-puncture spacing piece is a detachable piece.

In the embodiment of the application, the peripheral wall of the outer cylinder is provided with a spacer inserting groove, the cylinder cavity area of the cylinder cavity of the outer cylinder between the inner end of the puncture needle and the stopcock is communicated with the spacer inserting groove, and the anti-puncture spacing piece is inserted into the cylinder cavity area through the spacer inserting groove.

In an embodiment of the present application, there is provided an aqueous humor collection device including:
the outer cylinder comprises a puncture needle mounting end and a fluid collection tube mounting end;
the puncture needle is fixedly arranged at the installation end of the puncture needle and is provided with an inner end of the puncture needle positioned in the cylinder cavity of the outer cylinder and an outer end of the puncture needle positioned outside the cylinder cavity of the outer cylinder;
the fluid collection tube assembly comprises a fluid collection tube which is inserted into the outer cylinder cavity from the fluid collection tube mounting end and can be inserted and pulled in the axial direction, and a stopcock which is arranged in the outer cylinder cavity and is used for sealing the tube orifice of the fluid collection tube;
wherein, the outer peripheral wall of the liquid collection tube assembly is equipped with an annular groove and an annular limiting member provided by the annular groove, the radial outer end of the annular limiting member abuts against the inner peripheral wall of the outer cylinder, and smooth contact is formed between the radial outer edge of the stopcock and the inner wall of the outer cylinder, and there is a stopcock exhaust passage arranged along the extension direction of the outer cylinder cavity. The cylinder cavity of the outer cylinder is connected with the stopcock exhaust passage in the area of the cylinder cavity between the stopcock and the installation end of the puncture needle.

In the embodiment of the present application, the stopcock is provided with a plurality of stopcock exhaust passages extending along the axial direction of the outer cylinder cavity and sequentially spaced along the circumferential direction of the stopcock.

In the embodiment of the present application, a stopcock exhaust groove serving as a stopcock exhaust passage is formed on the outer peripheral wall of the stopcock, and the stopcock exhaust groove is provided with a groove opening toward the radially outer side.

In the embodiment of the application, the passage peripheral wall of the stopcock exhaust passage is a closed peripheral wall.

In the embodiment of the present application, the cross-section of the stopcock exhaust passage has a semicircular shape, a square shape, or an arc shape.

In the embodiment of the application, the stopcock comprises a stopcock central part for sealing the tube orifice of the fluid collection tube and a stopcock outer ring part which is positioned on the radial outer side of the stopcock central part and is provided with a stopcock exhaust passage, and the stopcock outer ring part is at least partially formed into a loose ventilation structure.

In the embodiment of the application, the end part of the stopcock is provided with a puncture guide blind hole for guiding the inner end of the puncture needle to penetrate, and the outer contour shape of the inner end of the puncture needle is matched with the shape of the puncture guide blind hole.

In the embodiment of the application, a peripheral wall exhaust gap is arranged between the fluid collection tube and the inner peripheral wall of the outer cylinder, and the cylinder cavity area of the cylinder cavity of the outer cylinder between the stopcock and the puncture needle mounting end, the stopcock exhaust passage and the peripheral wall exhaust gap are sequentially communicated.

In the embodiment of the application, the peripheral wall of the inner end of the puncture needle is provided with a peripheral wall water outlet hole communicated with the needle cavity of the puncture needle.

In the embodiment of the application, the inner end of the puncture needle is also provided with an axial water outlet hole which is axially communicated with the needle cavity, and the axial distance between the hole center of the peripheral wall water outlet hole and the hole center of the axial water outlet hole is not more than 3 mm.

In an embodiment of the present application, there is provided an aqueous humor collection device including:
the outer cylinder comprises a puncture needle mounting end and a fluid collection tube mounting end;
the puncture needle is fixedly arranged at the installation end of the puncture needle and is provided with an inner end of the puncture needle positioned in the cylinder cavity of the outer cylinder and an outer end of the puncture needle positioned outside the cylinder cavity of the outer cylinder;
the fluid collection tube is inserted into the cylindrical cavity of the outer cylinder from the mounting end of the fluid collection tube and can be inserted and pulled in the axial direction;
the stopcock is arranged in the cylinder cavity of the outer cylinder and is used to seal the nozzle of the fluid collection tube. The stopcock exhaust passage communicates with the cylinder cavity area of the outer cylinder cavity between the stopcock and the installation end of the puncture needle.

In the embodiment of the present application, the stopcock is provided with a plurality of stopcock exhaust passages extending along the axial direction of the outer cylinder cavity and sequentially spaced along the circumferential direction of the stopcock.

In the embodiment of the present application, a stopcock exhaust groove provided with a groove opening facing radially outward is formed on the outer peripheral wall of the stopcock as a stopcock exhaust passage.

In the embodiment of the application, the passage peripheral wall of the stopcock exhaust passage is a closed peripheral wall.

In the embodiment of the present application, the cross-section of the stopcock exhaust passage has a semicircular shape, a square shape, or an arc shape.

In the embodiment of the application, the stopcock comprises a stopcock central part for sealing the tube orifice of the fluid collection tube and a stopcock outer ring part which is positioned on the radial outer side of the stopcock central part and is provided with a stopcock exhaust passage, and the stopcock outer ring part is at least partially formed into a loose ventilation structure.

In the embodiment of the application, the end part of the stopcock is provided with a puncture guide blind hole for guiding the inner end of the puncture needle to penetrate, and the outer contour shape of the inner end of the puncture needle is matched with the shape of the puncture guide blind hole.

In the embodiment of the application, a peripheral wall exhaust gap is arranged between the fluid collection tube and the inner peripheral wall of the outer cylinder, and the cylinder cavity area of the cylinder cavity of the outer cylinder between the stopcock and the puncture needle mounting end, the stopcock exhaust passage and the peripheral wall exhaust gap are sequentially communicated.

In the embodiment of the application, an aqueous humor collection device is provided, which comprises an outer cylinder, a puncture needle and a fluid collection tube assembly, wherein the outer cylinder is provided with a puncture needle mounting end and a fluid collection tube mounting end; wherein, aqueous humor collection device also includes an anti-shaking structure for limiting the displacement of the fluid collection tube assembly along the radial direction of the outer cylinder cavity.

In this application embodiment, the fluid collection tube assembly still includes the supporting tube rack, the fluid collection tube is inserted into the inner cavity of the supporting tube rack. The anti-shaking structure includes a limiting protrusion protruding from the outer peripheral wall of the supporting tube rack or from the inner peripheral wall of the outer cylinder. The ends are respectively connected with the outer peripheral wall of the supporting tube rack and the inner peripheral wall of the outer cylinder.

In the embodiment of the present application, the fluid collection tube and the supporting tube rack are fixed by screw connection or snap connection.

In the embodiment of the application, the limiting protrusion is integrally formed on the outer peripheral wall of the fluid collection tube; or, the fluid collection tube assembly includes that the overcoat is in the tube kit on the periphery wall of fluid collection tube, the limiting protrusion sets up on the periphery wall of tube kit.

In an embodiment of the present application, the tube kit is an elastic tube kit.

In the embodiment of the application, the anti-shaking structure comprises a plurality of limiting protrusion which are sequentially arranged at intervals along the circumferential direction;
or the anti-shaking structure comprises a limiting protrusion which is formed by continuously extending along the circumferential direction, and an exhaust gap is formed between the limiting protrusion and the outer circumferential wall of the supporting tube rack or between the limiting protrusion and the inner circumferential wall of the outer cylinder.

In the embodiment of the application, the plurality of limiting protrusions sequentially arranged at intervals along the circumferential direction are all in a convex point shape.

In the embodiment of the application, the limiting protrusions which are formed by extending continuously along the circumferential direction are corrugated or helical teeth.

In an embodiment of the present application, there is provided an aqueous humor collection device including:
the outer cylinder is provided with a puncture needle mounting end and a fluid collection tube mounting end;
the puncture needle is inserted into the puncture needle mounting end and comprises a puncture needle outer end and a puncture needle inner end extending into the cylinder cavity of the outer cylinder;
the fluid collection tube assembly is inserted into the outer cylinder cavity from the fluid collection tube mounting end in a pluggable and movable manner and comprises a fluid collection tube and a stopcock used for sealing the tube orifice of the fluid collection tube in the outer cylinder cavity; wherein, a ventilation structure is formed between the fluid collection tube assembly and the inner peripheral wall of the outer cylinder, and the ventilation structure communicates with the cylinder cavity area of the outer cylinder cavity between the inner end of the puncture needle and the stopcock, the aqueous humor collection device further includes a limiting buffer structure for limiting the displacement of the fluid collection tube assembly in the radial direction and for forming contact damping between the fluid collection tube assembly and the inner peripheral wall of the outer cylinder.

In this application embodiment, the limiting buffer structure comprises a limiting protrusion protruding from the outer circumferential wall of the fluid collection tube assembly or from the inner circumferential wall of the outer cylinder, and two radial ends of the limiting protrusion are respectively connected with the outer circumferential wall of the fluid collection tube assembly and the inner circumferential wall of the outer cylinder.

In this application embodiment, the limiting buffer structure comprises a limiting protrusion follows the tube length direction of the fluid collection tube assembly sets at intervals or follows the tube length direction extends, the total span of the limiting protrusion followed tube length direction is not less than the half of fluid collection tube.

In the embodiment of the application, the limiting protrusion is integrally formed on the outer peripheral wall of the fluid collection tube; or, the fluid collection tube assembly includes that the overcoat is in the tube kit on the periphery wall of fluid collection tube, the limiting protrusion sets up on the periphery wall of tube kit.

In an embodiment of the present application, the tube kit is an elastic tube kit.

In the embodiment of the application, the limiting buffer structure comprises a plurality of limiting protrusion which are sequentially arranged at intervals along the circumferential direction, and gaps among the limiting protrusion formed the ventilation structure;
or, the limiting buffer structure includes along the continuous shaping of extending of circumference the limiting protrusion, the limiting protrusion with be formed with between the periphery wall of fluid collection tube assembly or the limiting protrusion with be formed with between the inner peripheral wall of cylinder outward as ventilation structure's exhaust clearance.

In the embodiment of the application, the plurality of limiting protrusions sequentially arranged at intervals along the circumferential direction are all in a convex point shape.

In the embodiment of the application, the limiting protrusions which are formed by extending continuously along the circumferential direction are corrugated or helical teeth.

In the embodiment of the application, the fluid collection tube assembly still includes the supporting tube rack that is used for fixed mounting the fluid collection tube, the limiting buffer structure is formed between the outer peripheral wall of the supporting tube rack and the inner peripheral wall of the outer cylinder.

In this application embodiment, the fluid collection tube and the supporting tube rack are fixed by screw connection or snap connection.

In an embodiment of the present application, an aqueous humor collection device includes:
the outer cylinder is provided with a puncture needle mounting end and a fluid collection tube mounting end;
the puncture needle is inserted into the puncture needle mounting end and comprises a puncture needle outer end and a puncture needle inner end extending into the cylinder cavity of the outer cylinder;
the fluid collection tube assembly is inserted into the outer cylinder cavity from the fluid collection tube mounting end in a pluggable and movable manner and comprises a fluid collection tube and a stopcock used for sealing the tube orifice of the fluid collection tube in the outer cylinder cavity; wherein, the coefficient of static friction between stopcock and the inner circumferential wall of outer cylinder is µ1, the stopcock with the coefficient of static friction between stopcock and the port of fluid collection tube is µ2, and satisfies: µ1 <µ2;
or the stopcock is arranged at intervals on the inner peripheral wall of the outer cylinder.

In this application embodiment, smooth contact is formed between the stopcock and the inner peripheral wall of the outer cylinder.

In this application embodiment, the aqueous humor collection device further comprises an anti-shaking structure for limiting the radial displacement of the fluid collection tube assembly along the cylindrical cavity of the outer cylinder.

In the embodiment of the application, the anti-shaking structure comprises an annular groove and an annular limiting member, the annular groove is formed in the outer circumferential wall of the fluid collection tube assembly, the annular limiting member is arranged circumscribing the annular groove, and the radially outer end of the annular limiting member abuts against the inner circumferential wall of the outer cylinder.

In this application embodiment, the groove side wall of the annular groove is formed with a groove vent hole which is through along the axial direction of the outer cylinder cavity, and the outer cylinder cavity is communicated with the groove vent hole in the cylinder cavity area between the stopcock and the puncture needle mounting end.

In this application embodiment, the fluid collection tube assembly includes supporting tube rack, the fluid collection tube is inserted into the inner cavity of the supporting tube rack, and the annular groove is provided on the outer peripheral wall of the supporting tube rack.

In the embodiment of the application, the fluid collection tube assembly still includes supporting tube rack, the fluid collection tube is inserted into the inner cavity of the supporting tube rack, the anti-shaking structure includes a limiting protrusion protruding from the outer peripheral wall of the supporting tube rack or from the inner peripheral wall of the outer cylinder, and the radial ends of the limiting protrusion are respectively connected to the outer peripheral wall of the supporting tube rack and the outer peripheral wall of the outer cylinder.

In the embodiment of the application, the aqueous humor collection device further comprises a needle seat for fixedly mounting the puncture needle, and the inner end of the needle seat is connected with the mounting end of the puncture needle in a quick plugging manner.

In the embodiment of the application, the aqueous humor collection device further comprises an anti-puncture spacing piece for isolating the inner end of the puncture needle from the stopcock along the axial direction of the cylindrical cavity of the outer cylinder, and the anti-puncture spacing piece is a detachable piece.

In the embodiment of the application, the peripheral wall of the outer cylinder is provided with a spacer inserting groove, and the cylinder cavity of the outer cylinder communicates with the spacer inserting groove in the area between the inner end of the puncture needle and the stopcock, the anti-puncture spacing piece is inserted into the cylinder cavity area through the spacer inserting groove.

In an embodiment of the present application, there is provided a method of collecting aqueous humor from a cornea, comprising:
the outer end of the puncture needle of the aqueous humor collection device is punctured into the anterior chamber of the eye;
pushing the rear end of a fluid collection tube assembly to enable a fluid collection tube in the fluid collection tube assembly to move towards the inner end of the puncture needle, so that the inner end of the puncture needle penetrates through a stopcock and enters the fluid collection tube;
allowing aqueous humor of the anterior chamber to flow through the puncture needle and into the fluid collection tube at a pressure differential between the intraocular pressure and the fluid collection tube;
withdrawing the aqueous humor collection device from the anterior chamber; and taking out the fluid collection tube assembly from the outer cylinder.

In an embodiment of the present application, the method comprises pushing the rear end of the fluid collection tube assembly to move the fluid collection tube in the fluid collection tube assembly towards the inner end of the puncture needle to pass the inner end of the puncture needle through the stopcock and into the fluid collection tube and include:
holding the aqueous humor collection device with a single hand and nudging the rear end of the fluid collection tube assembly with an index finger.

In an embodiment of the present application, the present invention provides a method for collecting aqueous humor from an eye of a subject, comprising:
inserting an outer end of a puncture needle of any of the aqueous humor collection devices provided according to the present invention into an anterior chamber of the eye, wherein a pressure within the fluid collection tube is lower than an intraocular pressure of the subject;
pushing the rear end of a fluid collection tube assembly to enable a fluid collection tube in the fluid collection tube assembly to move towards the inner end of the puncture needle, so that the inner end of the puncture needle penetrates through a stopcock and enters the fluid collection tube;
allowing aqueous humor of the anterior chamber of the eye to flow through the puncture needle and into the fluid collection tube at a pressure differential between the intraocular pressure and the negative pressure;
withdrawing the aqueous humor collection device from the anterior chamber; and and taking out the fluid collection tube assembly from the outer cylinder.

In an embodiment of the present application, the method comprises pushing the rear end of the fluid collection tube assembly to move the fluid collection tube in the fluid collection tube assembly towards the inner end of the puncture needle to pass the inner end of the puncture needle through a stopcock into the fluid collection tube and include:
holding the aqueous humor collection device with a single hand and nudging the rear end of the fluid collection tube assembly with an index finger.

In an embodiment of the present application, the method comprises selecting a negative pressure inside the fluid collection tube that is below an intraocular pressure of an eye of the subject.

In the embodiment of this application, providing a reagent holder, and the reagent holder includes the holder body and functional station provided on the holder body, and this functional station includes fluid holding tube insertion hole and elastic component, and the elastic component is configured to: when the fluid holding tube is inserted into the fluid holding tube insertion hole, the fluid holding tube is elastically moved in the direction of the central axis of the fluid holding tube insertion hole by elastically acting on the fluid holding tube.

In the embodiment of the application, the functional station further comprises a functional station outer sleeve, and the functional station outer sleeve extends downward from the inner peripheral wall of the fluid holding tube insertion hole in the direction of the central axis of the hole, the elastic component is provided in the sleeve inner cavity of the functional bit outer sleeve.

In the embodiment of the present application, the elastic component is a spring mechanism or an elastic medium.

In this application embodiment, the spring mechanism is located at the bottom of the inner cavity of the sleeve and can elastically move along the central axis of the hole, and during the elastic movement of the spring mechanism, at least part of the outer peripheral part of the spring mechanism slides along the inner peripheral wall of the inner cavity of the sleeve.

In the embodiment of the application, the elastic component is positioned at the bottom of the inner cavity of the sleeve, and a supporting concave position for supporting the bottom of the fluid holding tube is formed at the center of the top of the elastic component.

In the embodiment of the application, the reagent holder is a strip-shaped holder.

In the embodiments of the present application, the functional station includes a sample station, a reagent station, a light measuring station, a reaction station or a reserved station.

In an embodiment of the application, there is provided a test apparatus assembly comprising a testing apparatus, a fluid holding tube and a reagent holder according to any one of claims 1 to 7, the fluid holding tube being detachably inserted in the fluid holding tube insertion hole, the testing apparatus comprising a liquid drawing device configured to draw liquid from a lumen extending into the fluid holding tube.

In an embodiment of the application, the testing apparatus is arranged to stop when the liquid drawing device is subjected to a rigid impact.

In the embodiment of the application, the functional station further comprises a functional station outer sleeve, the functional station outer sleeve extends downwards from the inner peripheral wall of the fluid holding tube insertion hole along the direction of the central axis of the hole, the outer contour of the bottom of the fluid holding tube is in an inverted cone shape, the fluid holding tube penetrates through the fluid holding tube insertion hole to be inserted into the sleeve inner cavity of the functional station outer sleeve, the top end of the fluid holding tube is not higher than the orifice of the fluid holding tube insertion hole, and the outer diameter of the tube orifice of the fluid holding tube is the same as the inner diameter of the sleeve inner cavity.

In an embodiment of the present application, there is provided a testing apparatus assembly, including: the reagent holder is provided with a holder body and a functional station arranged on the holder body, and the functional station comprises a fluid holding tube insertion hole and an elastic component;
the fluid holding tube is detachably inserted in the fluid holding tube insertion hole and can elastically move along the central axis direction of the hole of the fluid holding tube insertion hole under the elastic action of the elastic component;
the testing apparatus is provided with a liquid drawing needle which is used for extending into the tube cavity of the fluid holding tube to draw liquid, the outer end face of the liquid drawing needle is formed into an inclined end face, and a liquid drawing port is formed in the inclined end face.

In this application embodiment, the liquid drawing needle extends along a straight line and comprises a needle body section and a needle head section which are connected with each other, the outer end of the needle head section is provided with the fluid drawing port, and the central axis of the needle body section is intersected with the central axis of the fluid drawing port.

In this application embodiment, an acute included angle formed by intersection of the central axis of the needle body section and the central axis of the fluid drawing port is α, and α satisfies: 0<α≤0.1°.

In this application embodiment, the inclined end surface is a curved surface or a flat surface.

In this application embodiment, the bottom wall in the cavity of the fluid holding tube is a plane wall or a curved wall.

In this application embodiment, the testing apparatus is arranged to stop when the liquid drawing needle is rigidly impacted.

In this application embodiment, the functional station further comprises a functional station outer sleeve, the functional station outer sleeve extends downwards from the inner peripheral wall of the fluid holding tube insertion hole along the direction of the central axis of the hole, and the elastic component is located in a sleeve inner cavity of the functional station outer sleeve.

In this application embodiment, the elastic component is a spring mechanism or an elastic medium.

In this application embodiment, the elastic component is located the bottom of sleeve inner chamber, the top center of elastic component is formed with the support concave position that is used for supporting the bottom of fluid holding tube, the bottom outline of fluid holding tube is the back taper, the shape that supports concave position match in the bottom outline of fluid holding tube.

In this application embodiment, the functional station is a sample station, a reagent station, a light measuring station, a reaction station or a reserved station.

In this application embodiment, providing a reagent holder, which comprises a holder body and a functional station disposed on the holder body, wherein the functional station comprises a fluid holding tube insertion hole and an elastic component, and the elastic component is configured to: under the condition that a fluid holding tube is inserted into the fluid holding tube insertion hole, the elastic force can act on the fluid holding tube, so that the fluid holding tube can elastically move along the direction of the central axis of the hole of the fluid holding tube insertion hole.

In this application embodiment, the functional station further comprises a functional station outer sleeve, the functional station outer sleeve extends downwards from the inner peripheral wall of the fluid holding tube insertion hole along the direction of the central axis of the hole, and the elastic component is located in a sleeve inner cavity of the functional station outer sleeve.

In the embodiment of this application, the elastic component is a spring mechanism or an elastic medium.

In the embodiment of this application, the spring mechanism is located at the bottom of the inner cavity of the sleeve and can elastically move along the central axis of the hole, and during the elastic movement of the spring mechanism, at least part of the outer peripheral part of the spring mechanism slides along the inner peripheral wall of the inner cavity of the sleeve.

In the embodiment of the application, the reagent holder further comprises a conversion socket which is detachably inserted into the fluid holding tube insertion hole, the conversion socket comprises a support sleeve part and an extension sleeve part which are connected up and down, sleeve cavities of the support sleeve part and the extension sleeve part are communicated up and down and jointly form a conversion insertion cavity, and the conversion insertion cavity is used for detachably inserting the fluid holding tube.

In the embodiment of the application, the functional station is a sample station, a reagent station, a light measuring station, a reaction station or a reserved station.

In the embodiment of the application, there is provided a test apparatus assembly comprising a testing apparatus, a fluid holding tube and foretell reagent holder, the fluid holding tube being detachably inserted in the fluid holding tube insertion hole, the testing apparatus comprising a liquid drawing device configured to draw liquid from a lumen extending into the fluid holding tube.

In an embodiment of the application, the testing apparatus is arranged to stop when the liquid drawing device is subjected to a rigid impact.

In the embodiment of the application, the functional station further comprises a functional station outer sleeve, the functional station outer sleeve extends downwards from the inner peripheral wall of the fluid holding tube insertion hole along the direction of the central axis of the hole, the outer contour of the bottom of the fluid holding tube is in an inverted cone shape, the fluid holding tube penetrates through the fluid holding tube insertion hole to be inserted into the sleeve inner cavity of the functional station outer sleeve, the top end of the fluid holding tube is not higher than the orifice of the fluid holding tube insertion hole, and the outer diameter of the tube orifice of the fluid holding tube is the same as the inner diameter of the sleeve inner cavity.

In the embodiment of the present application, the liquid drawing device is provided with a liquid drawing needle extending into the lumen of the fluid collection tube to take liquid, and the outer end surface of the liquid drawing needle is formed as an inclined end surface and the liquid drawing port is formed on the inclined end surface.

In an embodiment of the present application, an aqueous humor collection device includes:
the outer cylinder is provided with a puncture needle mounting end and a fluid collection tube mounting end;
the puncture needle is inserted into the puncture needle mounting end and comprises a puncture needle outer end and a puncture needle inner end extending into the cylinder cavity of the outer cylinder;
the fluid collection tube assembly is inserted into the outer cylinder cavity from the fluid collection tube mounting end in a pluggable and movable manner and comprises a fluid collection tube and a stopcock used for sealing the tube orifice of the fluid collection tube in the outer cylinder cavity; wherein, the coefficient of static friction between stopcock and the inner circumferential wall of outer cylinder is µ1, the stopcock with the coefficient of static friction between stopcock and the port of fluid collection tube is µ2, and satisfies: µ1 <µ2;
or the stopcock is arranged at intervals on the inner peripheral wall of the outer cylinder.

In this application embodiment, smooth contact is formed between the stopcock and the inner peripheral wall of the outer cylinder.

In this application embodiment, the aqueous humor collection device further comprises an anti-shaking structure for limiting the radial displacement of the fluid collection tube assembly along the cylindrical cavity of the outer cylinder.

In the embodiment of the application, the anti-shaking structure comprises an annular groove and an annular limiting member, the annular groove is formed in the outer circumferential wall of the fluid collection tube assembly, the annular limiting member is arranged circumscribing the annular groove, and the radially outer end of the annular limiting member abuts against the inner circumferential wall of the outer cylinder.

In this application embodiment, the groove side wall of the annular groove is formed with a groove vent hole which is through along the axial direction of the outer cylinder cavity, and the outer cylinder cavity is communicated with the groove vent hole in the cylinder cavity area between the stopcock and the puncture needle mounting end.

In this application embodiment, the fluid collection tube assembly includes supporting tube rack, the fluid collection tube is inserted into the inner cavity of the supporting tube rack, and the annular groove is provided on the outer peripheral wall of the supporting tube rack.

In the embodiment of the application, the fluid collection tube assembly still includes supporting tube rack, the fluid collection tube is inserted into the inner cavity of the supporting tube rack, the anti-shaking structure includes a limiting protrusion protruding from the outer peripheral wall of the supporting tube rack or from the inner peripheral wall of the outer cylinder, and the radial ends of the limiting protrusion are respectively connected to the outer peripheral wall of the supporting tube rack and the outer peripheral wall of the outer cylinder.

In the embodiment of the application, the aqueous humor collection device further comprises a needle seat for fixedly mounting the puncture needle, and the inner end of the needle seat is connected with the mounting end of the puncture needle in a quick plugging manner.

In the embodiment of the application, the aqueous humor collection device further comprises an anti-puncture spacing piece for isolating the inner end of the puncture needle from the stopcock along the axial direction of the cylindrical cavity of the outer cylinder, and the anti-puncture spacing piece is a detachable piece.

In the embodiment of the application, the peripheral wall of the outer cylinder is provided with a spacer inserting groove, and the cylinder cavity of the outer cylinder communicates with the spacer inserting groove in the area between the inner end of the puncture needle and the stopcock, the anti-puncture spacing piece is inserted into the cylinder cavity area through the spacer inserting groove.

In an embodiment of the present application, there is provided a method of collecting aqueous humor from a cornea, comprising:
the outer end of the puncture needle of the aqueous humor collection device is punctured into the anterior chamber of the eye;
pushing the rear end of a fluid collection tube assembly to enable a fluid collection tube in the fluid collection tube assembly to move towards the inner end of the puncture needle, so that the inner end of the puncture needle penetrates through a stopcock and enters the fluid collection tube;
allowing aqueous humor of the anterior chamber to flow through the puncture needle and into the fluid collection tube at a pressure differential between the intraocular pressure and the fluid collection tube;
withdrawing the aqueous humor collection device from the anterior chamber; and taking out the fluid collection tube assembly from the outer cylinder.

In an embodiment of the present application, the method comprises pushing the rear end of the fluid collection tube assembly to move the fluid collection tube in the fluid collection tube assembly towards the inner end of the puncture needle to pass the inner end of the puncture needle through the stopcock and into the fluid collection tube and include:
holding the aqueous humor collection device with a single hand and nudging the rear end of the fluid collection tube assembly with an index finger.

In an embodiment of the present application, the present invention provides a method for collecting aqueous humor from an eye of a subject, comprising:
inserting an outer end of a puncture needle of any of the aqueous humor collection devices provided according to the present invention into an anterior chamber of the eye, wherein a pressure within the fluid collection tube is lower than an intraocular pressure of the subject;
pushing the rear end of a fluid collection tube assembly to enable a fluid collection tube in the fluid collection tube assembly to move towards the inner end of the puncture needle, so that the inner end of the puncture needle penetrates through a stopcock and enters the fluid collection tube;
allowing aqueous humor of the anterior chamber of the eye to flow through the puncture needle and into the fluid collection tube at a pressure differential between the intraocular pressure and the negative pressure;
withdrawing the aqueous humor collection device from the anterior chamber; and taking out the fluid collection tube assembly from the outer cylinder.

In the embodiment of the application, the method comprises pushing the rear end of the fluid collection tube assembly to move the fluid collection tube in the fluid collection tube assembly towards the inner end of the puncture needle to pass the inner end of the puncture needle through the stopcock and into the fluid collection tube and include:
holding the aqueous humor collection device with a single hand and nudging the rear end of the fluid collection tube assembly with an index finger.

In an embodiment of the present application, a method includes: the negative pressure inside the fluid collection tube is selected to be lower than the intraocular pressure of the eye of the subject.

Although the embodiments of the present invention have been described in detail with reference to the accompanying drawings, the embodiments of the present invention are not limited to the specific details of the above embodiments, and various simple modifications may be made to the technical solutions of the embodiments of the present invention within the technical idea of the embodiments of the present invention, and these simple modifications all belong to the protection scope of the embodiments of the present invention.

It should be noted that, in the above embodiments, the various features described in the above embodiments may be combined in any suitable manner, and in order to avoid unnecessary repetition, the embodiments of the present invention do not describe any combination of the features.

In addition, any combination of various different implementation manners of the embodiments of the present invention can be made, and the embodiments of the present invention should also be regarded as the disclosure of the embodiments of the present invention as long as the combination does not depart from the spirit of the embodiments of the present invention.

## Claims

1. The reagent holder (9) comprises a holder body and functional station arranged on the holder body, wherein the functional station comprise a fluid holding tube insertion hole (91) and an elastic component, and the elastic component is set as follows: under the condition that a fluid holding tube (106) is inserted in the fluid holding tube insertion hole (91), the elastic force can act on the fluid holding tube (106) so that the fluid holding tube (106) can move elastically along the direction of the central axis of the hole of the fluid holding tube insertion hole (91).

2. The reagent holder according to claim 1, wherein the functional station further comprises a functional station outer sleeve (92), the functional station outer sleeve (92) extends downwards from the inner peripheral wall of the fluid holding tube insertion hole (91) along the direction of the central axis of the hole, and the elastic component is positioned in a sleeve inner cavity of the functional station outer sleeve (92).

3. The reagent holder according to claim 2, wherein the elastic component is a spring mechanism (93) or an elastic medium.

4. The reagent holder according to claim 3, wherein the spring mechanism (93) is located at the bottom of the inner cavity of the sleeve and is capable of moving elastically in the direction of the central axis of the hole, and during the elastic movement of the spring mechanism (93), the outer peripheral portion of the spring mechanism (93) slides at least partially against the inner peripheral wall of the inner cavity of the sleeve.

5. The reagent holder according to claim 1, wherein the reagent holder (9) further comprises a switching jack (914) detachably inserted into the fluid holding tube insertion hole (91), the switching jack (914) comprises a supporting sleeve part (914a) and an extending sleeve part (914b) which are connected up and down, the sleeve cavities of the supporting sleeve part (914a) and the extending sleeve part (914b) are communicated up and down and jointly form a switching insertion cavity, and the switching insertion cavity is used for detachably inserting the fluid holding tube (106).

6. The reagent holder of any one of claims 1-5, wherein the functional station comprise a sample station (95), a reagent station (98), a light measuring station (99), a reaction station (910), or a reserved station (912).

7. A testing apparatus assembly, comprising a testing apparatus (10), a fluid holding tube (106) and a reagent holder (9) according to any one of claims 1-6, wherein the fluid holding tube (106) is detachably inserted into the fluid holding tube insertion hole (91), and the testing apparatus (10) comprises a liquid drawing device which is set to draw liquid from the tube cavity extending into the fluid holding tube (106).

8. The testing apparatus assembly according to claim 7, wherein the testing apparatus (10) is set to stop when the liquid drawing device is subjected to a rigid impact.

9. The testing apparatus assembly according to claim 7, wherein the functional station further comprises a functional bit outer sleeve (92), the functional bit outer sleeve (92) extends downwards from the inner peripheral wall of the fluid holding tube insertion hole (91) along the direction of the central axis of the hole, the outer bottom contour of the fluid holding tube (106) is in an inverted cone shape, the fluid holding tube (106) passes through the fluid holding tube insertion hole (91) to be inserted into the sleeve inner cavity of the functional bit outer sleeve (92) and the top end of the fluid holding tube (106) is not higher than the orifice of the fluid holding tube insertion hole (91), and the outer diameter of the orifice of the fluid holding tube (106) is the same as the inner diameter of the sleeve inner cavity.

10. The testing apparatus assembly according to claim 7, wherein the fluid drawing device is provided with a liquid drawing needle (104) for taking liquid extending into a lumen of the fluid holding tube (106), an outer end surface of the liquid drawing needle (104) is formed into an inclined end surface (104a) and a liquid drawing port is set on the inclined end face (104a).

11. An aqueous humor collection device, comprising:
the outer cylinder (1) is provided with a puncture needle mounting end and a fluid collection tube mounting end which are positioned at two axial ends;
the puncture needle (2) is fixedly installed at the puncture needle mounting end and is provided with a puncture needle inner end positioned in the outer cylinder cavity and a puncture needle outer end positioned outside the outer cylinder cavity;
the fluid collection tube assembly (3) comprises a fluid collection tube (31) which is inserted into the outer cylinder cavity from the fluid collection tube mounting end and can be inserted and pulled in the axial direction, and a stopcock (33) which is set in the outer cylinder cavity and is used for sealing the tube orifice of the fluid collection tube;
wherein, the coefficient of static friction between stopcock (33) and the inner circumferential wall of outer cylinder is µ1, the coefficient of static friction between stopcock (33) and the fluid collection tube mouth of tube is µ2, and satisfies: µ1 <µ2;
or the stopcock (33) is set at intervals on the inner peripheral wall of the outer cylinder.

12. The aqueous humor collection device according to claim 11, wherein the stopcock (33) forms a smooth contact with the inner circumferential wall of the outer cylinder.

13. The aqueous humor collection device according to claim 11, further comprising an anti-shaking structure for limiting the radial displacement of the fluid collection tube assembly (3) along the cylindrical cavity of the outer cylinder.

14. The aqueous humor collection device according to claim 13, wherein the anti-shaking structure comprises an annular groove (35) formed on the outer circumferential wall of the fluid collection tube assembly (3) and an annular limiting member (37) provided around the annular groove (35), and a radially outer end of the annular limiting member (37) abuts against the inner circumferential wall of the outer cylinder.

15. The aqueous humor collection device according to claim 14, wherein the groove side wall of the annular groove (35) is formed with a groove vent hole (36) penetrating in the axial direction of the outer cylinder cavity communicating with the groove vent hole (36) in the cylinder cavity region between the stopcock (33) and the puncture needle mounting end.

16. The aqueous humor collection device according to claim 14, wherein the fluid collection tube assembly (3) comprises a supporting tube rack (32), the fluid collection tube (31) is inserted into a tube holder inner cavity of the supporting tube rack (32), and the annular groove (35) is provided on an outer circumferential wall of the supporting tube rack (32).

17. The aqueous humor collection device according to claim 13, wherein the fluid collection tube assembly (3) further comprises a supporting tube rack (32), the fluid collection tube (31) is inserted into a tube rack inner cavity of the supporting tube rack (32), the anti-shaking structure comprises a limiting protrusion (6) protruding from an outer circumferential wall of the supporting tube rack (32) or from an inner circumferential wall of an outer cylinder, and two radial ends of the limiting protrusion (6) are respectively connected with the outer circumferential wall of the supporting tube rack (32) and the inner circumferential wall of the outer cylinder.

18. The aqueous humor collection device according to claim 11, further comprising a needle seat (4) for fixedly mounting the puncture needle (2), wherein a quick stopcock connection is formed between the inner end of the needle seat (4) and the mounting end of the puncture needle.

19. The aqueous humor collection device according to any one of claims 11 to 18, further comprising an anti-puncture spacing piece (5) for isolating the inner end of the puncture needle from the stopcock (33) in the axial direction of the outer cylinder cavity, wherein the anti-puncture spacing piece (5) is a detachable member.

20. The aqueous humor collection device according to claim 19, wherein the peripheral wall of the outer cylinder (1) is provided with a spacer inserting groove (11), the outer cylinder cavity communicates with the spacer inserting groove (11) at a chamber region between the inner end of the puncture needle and the stopcock (33), and the anti-puncture spacing piece (5) is inserted into the chamber region through the spacer inserting groove (11).

21. A method of collecting aqueous humor from a cornea, comprising:
inserting the outer end of the puncture needle (2) of the aqueous humor collection device according to any one of claims 11 to 20 into the anterior chamber of the eye;
pushing the rear end of a fluid collection tube assembly (3) to enable a fluid collection tube (31) in the fluid collection tube assembly (3) to move towards the inner end of the puncture needle (2) so as to enable the inner end of the puncture needle to penetrate through a stopcock (33) to enter the fluid collection tube (31);
passing aqueous humor of the anterior chamber through the puncture needle (2) and into the fluid collection tube (31) at a pressure differential between the intraocular pressure and the fluid collection tube;
withdrawing the aqueous humor collection device from the anterior chamber; and
taking out the fluid collection tube assembly (3) from the outer cylinder(1).

22. The method according to claim 21, wherein pushing the rear end of the fluid collection tube assembly (3) to move the fluid collection tube (31) in the fluid collection tube assembly (3) towards the puncture needle inner end of the puncture needle (2) to pass the puncture needle inner end through a stopcock (33) into the fluid collection tube (31) comprises:
holding the aqueous humor collection device by a single hand, and slightly pushing the rear end of the fluid collection tube assembly (3) by an index finger.

23. A method for collecting aqueous humor from an eye of a subject, comprising:
piercing the outer end of the puncture needle (2) of an aqueous humor collection device of any one of claims 11 to 20 into the anterior chamber of an eye, wherein the pressure in the collection tube (31) is lower than the intraocular pressure of the subject;
pushing the rear end of a fluid collection tube assembly (3) to enable a fluid collection tube (31) in the fluid collection tube assembly (3) to move towards the inner end of the puncture needle (2) so as to enable the inner end of the puncture needle to penetrate through a stopcock (33) to enter the fluid collection tube (31);
allowing aqueous humor of the anterior chamber of the eye to pass through the puncture needle (2) and flow into the fluid collection tube (31) at a pressure differential between the intraocular pressure and the negative pressure;
withdrawing the aqueous humor collection device from the anterior chamber; and
taking out the fluid collection tube assembly (3) from the outer cylinder (1).

24. The method according to claim 23, wherein pushing the rear end of the fluid collection tube assembly (3) to move the fluid collection tube (31) in the fluid collection tube assembly (3) towards the puncture needle inner end of the puncture needle (2) to pass the puncture needle inner end through a stopcock (33) into the fluid collection tube (31) comprises:
holding the aqueous humor collection device by a single hand, and slightly pushing the rear end of the fluid collection tube assembly (3) by an index finger.

25. The method according to claim 23 or 24, further comprising selecting a negative pressure inside the fluid collection tube (31) which is lower than the intraocular pressure of the eye of the subject.
